# EUROPEAN PATENT APPLICATION

(11) **EP 3 578 138 A1**
(43) Date of publication of application: **11.12.2019**
(21) Application number: 19173628.9
(22) Date of filing: 21.03.2017
(51) Int. Cl.: A61F 2/24

(54) **VASCULAR VALVED PROSTHESIS AND MANUFACTURING METHOD**

(30) Priority: 22.03.2016 EP 16161702
(62) Divisional of application: 17715062.0
(71) Applicant: ASSISTANCE PUBLIQUE, HOPITAUX DE PARIS, 75004 Paris (FR); Statice, 25000 Besancon (FR); UNIVERSITE PARIS DESCARTES, 75006 Paris (FR)
(72) Inventor: KALFA, David, Long Island City, NY 11104 (US); MENASCHÉ, Philippe, 75006 Paris (FR); POUPONNEAU, Pierre, 25000 Besançon (FR); LEONARD, Clément, 25000 Besançon (FR); PERROT, Sébastien, 25000 Besançon (FR)
(74) Representative: Gevers & Orès

(57) **Abstract**

The invention concerns a fully biodegradable vascular valved prosthesis, preferably allowing tissue regeneration and growth potential, comprising an electrospun inner conduit having a proximal end and a distal end and which is disposed within an electrospun outer conduit having a proximal end and a distal end, wherein the inner conduit is attached to the outer conduit such as to function as a valve allowing unidirectional flow of a fluid through said outer conduit from the outer conduit's proximal to distal end, wherein the proximal end of said inner conduit is attached circumferentially along the inside of the outer conduit towards the proximal end of the outer conduit to form a circumferential commissure, wherein the distal end of said inner conduit is attached to the inside of the outer conduit towards the distal end of said outer conduit to form focal commissures at one or more discrete positions, wherein the focal commissures may extend longitudinally from the distal end of the inner conduit towards the proximal end of the inner conduit.

## Description

### Field of the invention

The present invention relates to a fully biodegradable vascular valved prosthesis, allowing tissue regeneration and growth potential, a mandrel for electrospinning said vascular valved prosthesis, and a method for electrospinning said vascular valved prosthesis.

### Background to the invention

Vascular, valvar and cardiac diseases are characterized by an abnormal condition of the blood vessels or valves. These diseases often result in malformations such as lesions found in the heart's valvar system or to the vascular system connected thereto. Advanced stages may cause severe disability to the patient and even create life-threatening conditions through restriction or reduction of the blood flow to important organs. Treatment of the malformations usually involves a surgical correction to repair the right ventricular outflow track (RVOT), i.e. a major cardiac output channel which is involved in over half of congenital heart disease pathology. Examples of malformations include pulmonary atresia, persistent truncus arteriosus, vascular dispositioning with a pulmonary stenosis, tetralogy of Fallot, etc.; and also some procedures, such as the Ross procedure or pulmonary autograft, involve similar surgical corrections.

When one of the body's natural valves malfunctions, there exist prosthetic solutions designed to improve the cardiac output and recover a stable medical condition. For example, a mechanical or natural valve prosthesis connected to ducts serving as vascular tubes may be implanted to partially bypass, or completely replace a malformation obstructing the blood flow.

Current solutions mostly allow for a suboptimal, superficial correction of observed malformations resulting in several disadvantages. Particularly, their implantation procedure carries increased risks of infection, stenosis and/or calcification of the tube giving rise to fibrosis, aneurysms, regurgitation and/or stenosis of the valve.

Moreover, these current solutions, especially mechanical prostheses, are crafted from materials foreign to the human body (e.g., GoreTex®, Dacron®, etc.), which are likely to degenerate more or less rapidly depending on the patient's response and require the patient to take anticoagulation drugs for the remainder of his life. Further degeneration of the prosthetic solutions is then accompanied by a deterioration of the surgical corrections performed on the right ventricular outflow track, forcing the patient to redo the surgical procedure. Therefore, because of their size and implications, these prostheses are considered very unsuitable for children.

Although artificial materials may comprise a material strength above that of a natural artery, they may inadvertently prevent cell adhesion and tissue regeneration, which could otherwise prove beneficial for the safety and recovery rate of a patient. Since the current solutions use these artificial materials, the affected patients are forced to undergo numerous surgical re-interventions during their recovery process to maintain their stable medical condition by proactively preventing any degeneration. Each of these numerous re-interventions then further increases the risk for diseases, such as haemorrhagic syndrome, stroke, coronary artery disease, arrhythmias, conduction issues, or hospital-acquired infections; thereby significantly contributing to increased mortality rates, particularly for children.

Another solution may be found by extracting biological valves from animals; however, these valves may be undesirable because of their degeneration with time, their limited supply and for ethical reasons. Ideally, a vascular valved prosthesis manufactured from materials that may promote cell adhesion and tissue regeneration would provide an optimal solution. Additionally, it would also show complete biodegradability to be regenerated by surrounding tissue, thus evolving into a living, autologous valved vessel. However, achieving the inclusion of biocompatible materials within a product while also maintaining a high material and structural quality has proven difficult, in particular for complex tissue architectures.

Accordingly, there is a need to develop a more reliable and durable prosthesis product. There is a need for this product to retain good mechanical properties which may withstand the frequent patient movement, heart beating, and high fluidic pressures to which it will be subjected. There is also a need to develop a product which may be compatible with (young) children, especially children suffering from congenital heart disease affecting the right ventricular outflow track. There is also a need for a completely biodegradable product which may improve the tissue regeneration rate of the host.

Complicated arterial or vascular sections, for example those surrounding a pulmonary or aortic valve, may show a branching closely associated with further structural features, such as a valve. For those sections a standard tubular vascular prosthesis becomes less reliable as it may be difficult, or even impossible, to connect all arterial branches with a single tube. The medical choice may then be reconsidered to alternative shapes, such as bended prosthesis; however, these may result in a less reliable structural and material integrity over extended periods of time. For these reasons, incorporating a sufficiently sturdy, yet still elastic enough splitting region into a vascular prosthesis and in particular a vascular valved prosthesis has proven difficult.

Accordingly, there is a need for a product that may better mimic the shape of a natural artery, including branches, while also maintaining a high material and structural reliability. There is also a need for a product that may reliably combine all the above-described needs, such as compatibility with children and biodegradability, while also mimicking the shape of a natural artery. Additionally, there is a need for a method to make this product reliably.

A viable production method may be found in electrospinning, i.e., an electrostatic fiber fabrication technique method compatible with biomaterials. As its core concept, it involves the spinning of fibres around a mandrel or scaffold into the shape of choice. Using this technique a vascular valved prosthesis may be engineered with a nanoscale resolution and porosity similar to the natural extracellular matrix. However, one difficulty faced by this technique is found in the removal of the product, i.e., the electrospun prosthesis, from the mandrel. Generally after electrospinning the prosthesis sticks to the mandrel. Hence, to remove it mechanical forces need to be applied which can damage the prosthesis and/or its components. During forced removal the prosthesis will fumble up or crease and form folds along its surface. These deformations may degrade the structural and material integrity of the prosthesis prior to implant, thereby greatly diminishing the reliability and reproducibility of any prosthesis manufactured using electrospinning. Thus, a product that may be removed reliably from a mandrel without any deformations could have a much improved quality in comparison.

There exist several suboptimal solutions to avoid applying excessive force or friction on the prosthesis which add additional, time-consuming manufacturing steps. For example, a degradable sacrificial layer may be used between the mandrel and the prosthesis. However, to remove this sacrificial layer, the prosthesis has to be placed in a solution that may negatively affect biological materials or even prematurely initiate biodegradation. Alternatively, a sacrificial layer may be deposited consisting of materials with a lower electrical conductivity than the mandrel material, although, this in turn makes the electrospinning of a thick, i.e., above 100 µm, prosthesis very challenging and thus inaccurate. Another strategy is to wrap a mandrel with a metallic wire or foil, such as aluminium, that can be removed after the electrospinning process. However, this inadvertently leads to size variations of the prosthesis due to an unwanted excess space between the prosthesis and the mandrel, causing both the repeatability and reliability of the method to suffer. Reliably removing the prosthesis has so far proven difficult, yet by addressing this problem not only a faster, but also a more reliable production method would be obtained that may directly result in an improved product quality.

As such, there is a need in the art for a product comprising an artificial valve within a customizable vascular valved prosthesis that may combine a high structural and a material reliability, i.e., strong enough to withstand a high workload, with a shape better suited for mimicking natural arteries for improved functionality, yet that may potentially also incorporate biologically active materials. Additionally, there is a need for a vascular valved prosthesis to show complete biodegradability that may be regenerated by surrounding tissue and thus may evolve into living, autologous valved vessel. This is particularly needed for (young) children for whom the vascular valved prosthesis should feature a growth potential matching that of the child allowing for additional growth; thus preventing the need for multiple, subsequent operations to replace the previously implanted valve by one with a larger diameter. Additionally, there is a need for a valved vascular prosthesis comprising a T-shaped bifurcation that may achieve the reconstruction of additional arterial branches, such as the pulmonary artery branches. Additionally, there is a need for a method that may produce such a product in a quick, reliable manner. There is also a need to allow the product to be detached without detrimental structural damage; thereby possibly obtaining a product with an improved quality in comparison. The present invention serves to answer one or more of the above discussed problems in one or more aspects of the invention.

### Summary of the invention

A first aspect relates to a biodegradable electrospun vascular valved prosthesis (100) comprising an electrospun inner conduit (200) having a proximal end (P) and a distal end (D), and which is disposed within an electrospun outer conduit (300) having a proximal end and a distal end, wherein the inner conduit (200) is attached to the outer conduit (300) such as to function as a valve allowing unidirectional flow of a fluid through said outer conduit (300) from the outer conduit's proximal to distal end. The outer conduit may further be attached to a T-shaped conduit (400) to form a biodegradable electrospun vascular valved prosthesis (100) comprising a bifurcation (450), such as advantageously suitable for reconstruction of the pulmonary artery branches.

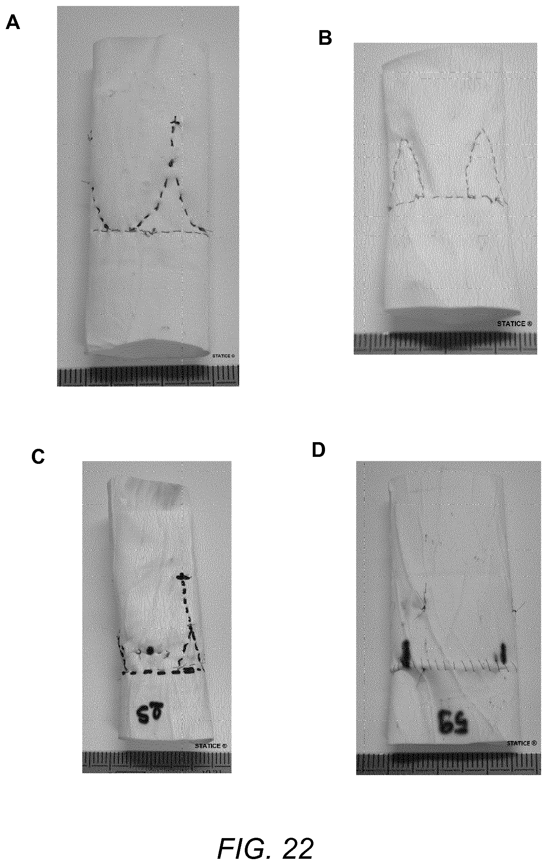

The proximal end of the inner conduit (200) may be disposed within the outer conduit (300) and subsequently attached along the inside of the outer conduit (300) towards the proximal end of the outer conduit (300) to form a circumferential commissure. The attachment may be performed using a variety of methods, such as sutures, staples, glue, welds (laser, vibration, ultrasonic, induction, high frequency) or a combination thereof. Next, the distal end of the inner conduit may be attached to the inside of the outer conduit towards the distal end of said outer conduit to form focal commissures (250) at one or more discrete positions, preferably two or more discrete positions, such as three discrete positions, wherein the focal commissures (250) may extend longitudinally from the distal end of the inner conduit towards the proximal end of the inner conduit, such as for instance depicted in Figure 22. Preferably, the focal commissures are equidistantly spaced. Preferably, the focal commissures are coplanar.

It will be understood that the prosthesis according to the invention is composed of or comprises a separate inner conduit and outer conduit, wherein the inside surface of the outer conduit is fixed to the outside surface of the inner conduit only at discrete locations (i.e. circumferentially along the proximal end of the inner conduit and at the focal commissures (which may be elongated along the proximal-distal axis) at the distal end of the inner conduit). Such attachment allows the non-attached portions of the inner conduit to function as cusps. The inner conduit is thus not attached to the outer conduit over the entire surface of the conduit.

Attachment of the inner tube at its distal end at the (longitudinally extended) focal/discrete commissures results in a more robust valve structure having improved functionality and allows for greater design flexibility. This is of particular importance for electrospun materials. On the other hand, connection of the distal end of the inner conduit by means of focal commissures creates cusps without the need to provide pre-formed cusps, such as for instance by means of specifically designed molds to obtain pre-formed cusps. In such way, mold design is greatly simplified. According to the invention, the inner conduit and the outer conduit are separately electrospun. Accordingly, the outer conduit is not directly electrospun on the inner conduit. In case of a T-shaped prosthesis, also the distal T-shaped portion of the prosthesis is separately electrospun. All parts of the prosthesis are connected to each other as described herein after electrospinning. This has the advantage that the inner and outer conduit can have different properties, such as degradation rate, polymer types, etc, which may differentially impact the mechanical or other properties of the inner and outer conduit. In case for instance the outer conduit would be electrospun directly upon the inner conduit, compatible polymers would need to be used in order to achieve good cohesion between outer and inner conduit such that adequate functionality is ensured. The provision of separate outer and inner conduits according to the present invention bypasses the need for compatible polymers and thus allows more flexibility.

The manufacturing of an T-shaped outer conduit in one time by electrospinning with one mandrel appears extremely challenging for several reasons: (i) rotation problems can occur due to the asymmetric shape of the conduit, (ii) it is challenging to remove the mandrel without damaging the T-shaped tube. Consequently, in order to manufacture by electrospinning with a simple system, according to certain embodiments of the invention, a T-shaped valved prosthesis is manufactured in different parts and then assembled together. Advantageously, such prosthesis can be manufactured with the mandrel according to the invention as described herein.

Along the section where the inner conduit is disposed, the outer conduit may further comprise protrusions (350) that for instance resemble sinus-like structures.

Thus, upon disposing the inner conduit (200) within the outer conduit (300) and subsequently attaching said conduits to each other, a valve-like functionality may be obtained resembling for instance that of the sinus of Valsalva, i.e., sinuses of the aorta or the pulmonary artery. During systole the inner conduit (200) may allow the circulation of fluid in one direction, namely from the proximal (P) end towards the distal (D) end; however, during diastole the inner conduit (200) may prevent the circulation of fluid in the opposite direction. Optionally, a T-shaped conduit (400) element may be fixed to the outer (300) conduit in the direction of the circulating fluid, similar to pulmonary artery branches.

The vascular valved prosthesis (100) may be, at least partially, formed from or coated or impregnated with a biologically active compound, preferably selected from peptides, growth factors, biological hydrogels (such as gelatin) and/or stem cells (such as adipose-derived stem cells). The vascular valved prosthesis (100) may be, at least partially, multi-layered wherein the layers comprise a fibrous network of microfibers and/or nanofibers.

A second aspect relates to a mandrel (600, 700) for electrospinning for instance the biodegradable electrospun vascular valved prosthesis (100) as described herein. The mandrel (600, 700) may comprise multiple components, wherein at least a part of the mandrel (600, 700) is configured to collapse with relation to the vascular valved prosthesis (100) once formed on the mandrel, i.e., at least a part of the mandrel may be folded or broken down inward upon removal of at least one mandrel fixation.

The exact components may vary depending on the conduit being electruspun, e.g., the inner conduit (200), the outer conduit (300) or the T-shaped conduit (400). Typically, the mandrel comprises at least one cylindrical-shaped central mandrel core (620, 720) and at least two shell pieces (640, 740) which when assembled form a cylindrical shaft or tube surrounding at least part of the mandrel core (620, 720).The shell pieces (640, 740) may be affixed to the core using one or more fixation means (660, 760). Additionally, at least one outer end of the mandrel core comprises a motor fixation means (670) to connect the mandrel (600, 700) to an electrospinning setup. Once everything is in place a conduit (200, 300, 400) may be electrospun onto and surrounding the shell pieces (640, 740).

After electrospinning, the mandrel fixation (660, 760) means securing the shell pieces (640, 740) to the mandrel core (620, 720) may be loosened or removed, such that the shell pieces (640, 740) become detached from the mandrel core (620, 720). This allows removal of the mandrel core (620, 720) and/or fixation means, for instance by sliding out from within the surrounding shell pieces (640, 740), which during removal may remain in place. Once the core (620, 720) is removed the structural support for the shell pieces (640, 740) is lost so that they may collapse inwardly. When collapsing, the shell pieces (640, 740) do not form a firm contact with the surrounding electrospun conduit (200, 300, 400) and may therefore easily be removed out from the prosthesis without causing structural or frictional damage, and/or without the need for using solvents or the like.

In a third aspect of the invention, the invention comprises the use of the mandrel for electrospinning, such as for instance electrospinning a biodegradable vascular valved prosthesis. In a fourth aspect of the invention, the invention comprises the method for manufacturing a prosthesis, such as for instance a biodegradable vascular valved prosthesis as defined herein, with the mandrel according to the invention.

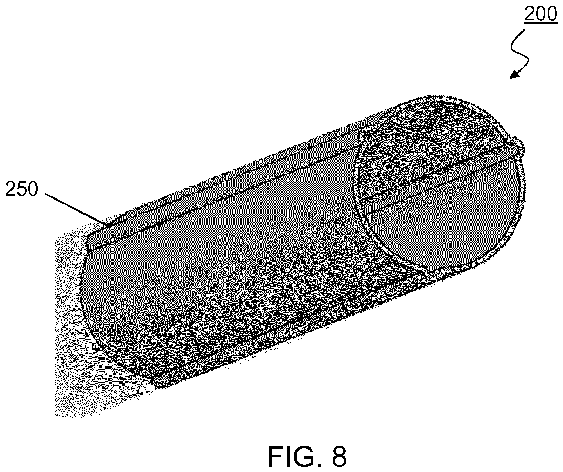

The present invention is in particular captured by numbered statements 1 to 49 below.
1. A (fully) biodegradable vascular valved prosthesis, comprising an electrospun inner conduit having a proximal end and a distal end and which is disposed within an electrospun outer conduit having a proximal end and a distal end, wherein the inner conduit is attached to the outer conduit such as to function as a valve allowing unidirectional flow of a fluid through said outer conduit from the outer conduit's proximal to distal end.
2. The prosthesis according to statement 1, which is bifurcated, preferably T-shaped.
3. The prosthesis according to statement 1 or 2, wherein said outer conduit comprises at least one radially outward protrusion; in case of a bifurcated or T-shaped prosthesis the protrusion(s) are located on the trunk of the prosthesis.
4. The prosthesis according to any of statements 1 to 3, wherein the proximal end of said inner conduit is attached circumferentially along the inside of the outer conduit towards the proximal end of the outer conduit to form a circumferential commissure; wherein the distal end of said inner conduit is attached to the inside of the outer conduit towards the distal end of said outer conduit to form focal commissures at one or more discrete positions, preferably two or more discrete positions, such as three discrete positions, wherein the focal commissures may extend longitudinally from the distal end of the inner conduit towards the proximal end of the inner conduit; optionally the focal commissures are equidistantly spaced; optionally the focal commissures are coplanar; in case of a bifurcated or T-shaped prosthesis the inner conduit is provided on the trunk of the prosthesis.
5. The prosthesis according to any of statements 1 to 4 for pulmonary valve replacement or aortic valve replacement.
6. The prosthesis according to any of statements 3 to 5, wherein said outer conduit comprises three coplanar radially equidistally spaced outward protrusions, and wherein the distal end of said inner conduit is attached at three equidistally spaced discrete positions to the inside of the outer conduit along longitudinal lines separating the protrusions.
7. The prosthesis according to any of statements 2 to 6, wherein a T-shaped conduit is attached along the distal end of said outer conduit.
8. The prosthesis according to any of statements 1 to 7, further comprising a biologically active compound, preferably selected from peptides, growth factors, biological hydrogels (such as gelatin) and/or stem cells (such as, but not exclusive of, adipose-derived stem cells).
9. The prosthesis according to any of statements 4 to 8, wherein biodegradability, i.e. rate of biodegratation, of the distal end of the commissures is faster than biodegradability of the proximal end of the commissures.
10. The prosthesis according to any of statements 1 to 9, wherein biodegradability, i.e. rate of biodegratation, of the inner conduit is slower than biodegradability of the outer conduit.
11. The prosthesis according to any of statements 1 to 10, wherein the inner and/or outer conduit(s) is/are multilayered.
12. The prosthesis according to any of statements 1 to 11, characterized in that the inner conduit has:
   - an elastic regimen of at least 40%, preferably at least 60%;
   - a Young's modulus in the circumferential direction ranging from 5 to 100 MPa and a Young's modulus in the radial direction ranging from 0.5 to 15 MPa; and
   - a Lagrangian strain in the circumferential direction ranging from 0.1 to 0.4 MPa and a Lagrangian strain in the radial direction ranging from 0.6 to 0.9 MPa.
13. The prosthesis according to any of statements 1 to 12, characterized in that the outer conduit has:
   - a swelling ratio ranging from 60 to 97%; and
   - a Young's modulus ranging 0.01 to 1 MPa.
14. The prosthesis according to any of statements 1 to 13, wherein the outer conduit and the inner conduit comprises a fibrous network comprising microfibers and/or nanofibers, preferably wherein the diameter of the fibers ranges between at least 0.2 to at most 3 µm, more preferably between 0.5 and 1.5 µm.
15. The prosthesis according to any of statements 1 to 14, wherein the inner tube has at least one region having a higher wall thickness at the distal end compared to the wall thickness at the proximal end.
16. The prosthesis according to any of statements 1 to 15, wherein the inner conduit is circumferentially or longitudinally reinforced, preferably by biodegradable structures.
17. The prosthesis according to any of statements 1 to 16, wherein said inner conduit and said outer conduit are made by electrospinning of biodegradable aliphatic polyesters and/or biodegradable polyurethanes.
18. The prosthesis according to any of statements 1 to 17, wherein said inner conduit is attached to said outer conduit by sutures, staples, glue, welds (laser, vibration, ultrasonic, induction, high frequency) or a combination thereof.
19. The prosthesis according to any of statements 1 to 18, wherein said inner conduit is attached to said outer conduit by sutures, glue or staples made from a biodegradable material.
20. The prosthesis according to any of statements 1 to 19, wherein
   - the diameter of the inner and outer conduits range between 15 and 25 mm;
   - the length of the inner conduit ranges between 15 and 30mm;
   - the length of the outer conduit ranges between 50 and 70 mm; and
   - the external circumferential diameter of the protrusions ranges between 25 and 35 mm.
21. The prosthesis according to any of statements 4 to 20, wherein the circumferential commissure is coplanar with the most proximal end of the protrusions.
22. The prosthesis according to any of statements 1 to 21, wherein the inner conduit when implanted in a vascular structure degrades in a period ranging from 6 to 30 months, such as 18 to 30 months and/or wherein the outer conduit when implanted in a vascular structure degrades in a period ranging from 6 to 18 months; preferably the degradation rate of the inner conduit does not exceed 12 months.
23. The prosthesis according to any of statements 1 to 22, wherein the inner conduit has a wall thickness ranging from 0.05 to 0.3 mm, such as 0.08 to 0.12 mm; preferably 0.150 to 0.250 mm, preferably (about) 0.200 mm.
24. The prosthesis according to any of statements 1 to 23, wherein the outer conduit has a wall thickness ranging from 0.1 to 1.2 mm, such as 0.5 mm to 1.2 mm, such as 0.3 to 0.7 mm.
25. The prosthesis according to any of statements 1 to 24, wherein the inner and/or outer conduit display anisotropic fiber orientation, wherein the anisotropic ratio is preferable at least 2:1, more preferably at least 30:1.
26. The prosthesis according to any of statements 1 to 25, wherein the inner conduit and/or outer conduit have a porosity ranging from 40 to 90%.
27. The prosthesis according to any of statements 1 to 26, wherein the inner conduit has a pore size (i.e. mean diameter) ranging from 0.3 to 5 µm and/or wherein the outer conduit has a pore size ranging from 20 to 40 µm
28. A mandrel for electrospinning, comprising a cylindrical mandrel core, one or more fixation means, and two or more shell pieces; wherein
   - said fixation means are configured for attaching said or more shell pieces on or to said mandrel core such as to form a cylindrical mandrel shell circumferentially encapsulating at least part of said mandrel core or a cylindrical mandrel shell affixed to said mandrel core;
   - said mandrel core and/or said fixation means are configured for being slidably removable from said mandrel, in particular without friction; and
   - said two or more shell pieces are configured for collapsing radially inward upon mandrel core and/or fixation means removal from said mandrel.
29. The mandrel according to statement 28, wherein said mandrel is for electrospinning a vascular prosthesis, preferably a biodegradable vascular prosthesis.
30. The mandrel according to statement 28 or 29, further comprising one or more radially extending protrusions, wherein said protrusions are configured for collapsing radially inward upon mandrel core and/or fixation means removal from said mandrel.
31. The mandrel according to any of statements 28 to 30, wherein the diameter of said mandrel shell is at least 5% larger than the diameter of said mandrel core, preferably at least 10% larger.
32. The mandrel according to any of statements 28 to 31, wherein said mandrel is bifurcated, preferably T-shaped.
33. The mandrel according to statement 32, wherein said mandrel comprises a mandrel trunk which is slidably affixed around said mandrel core such as to obtain a bifurcation, preferably a T-shape.
34. Use of the mandrel according to any of statements 28 to 33 for electrospinning.
35. The use according to statement 34 for electrospinning a vascular prosthesis, preferably a biodegradable vascular prosthesis.
36. The use according to statement 34 or 35 for electrospinning the prosthesis according to any of statements 1 to 27.
37. Method for manufacturing a vascular prosthesis, preferably a prosthesis according to any of statements 1 to 27, comprising the step of electrospinning a vascular prosthesis, preferably a biodegradable vascular prosthesis, using the mandrel according to any of statements 28 to 33.
38. The method according to statement 37, wherein said vascular prosthesis is suitable for pulmonary valve replacement or aortic valve replacement, comprising the steps of:
   - electrospinning an inner conduit having a distal end and a proximal end;
      electrospinning an outer conduit having a distal end and a proximal end, and comprising three coplanar radially equidistally spaced outward protrusions;
   - attaching the proximal end of said inner conduit circumferentially along the inside of the outer conduit towards the proximal end of the outer conduit to form a circumferential commissure;
   - attaching the distal end of said inner conduit at three equidistally spaced discrete positions to the inside of the outer conduit along longitudinal lines separating the protrusions;
   - electrospinning a T-shaped conduit having a trunk and lateral arms extending therefrom;
   - attaching the distal end of said outer conduit to the trunk of said T-shaped conduit; wherein said inner conduit is attached to the outer conduit such as to function as a valve allowing unidirectional flow of a fluid through said outer conduit from the outer conduit's proximal to distal end.
39. The prosthesis according to any of statements 1 to 26, which is biofunctionalized.
40. The prosthesis according to any of statements 1 to 26 or 39, made of a polymer or a blend of polymers compatible with exogenous cell seeding and/or featuring a biologically active signaling mechanism to drive endogenous cells inside and around the scaffold to achieve a fully autologous conduit.
41. The prosthesis according to any of statements 1 to 26 or 39 to 40, wherein the outer conduit comprises a proximal circumferential reinforcement, such as a reinforcement ring, preferably at or near the proximal end of the inner conduit, preferably wherein the reinforcement is biodegradable.
42. The prosthesis according to claim 41, wherein the reinforcement is disposed on the inside of the outer tube.
43. The prosthesis according to claim 41, wherein the reinforcement is disposed on the outside of the outer tube.
44. The prosthesis according to any of statements 1 to 26 or 39 to 43, wherein the distal end of the inner conduit has an increased wall thickness compared to the proximal end of the inner conduit.
45. The prosthesis according to any of statements 1 to 26 and 39 to 44, wherein the inner conduit comprises one or more longitudinal or circumferential reinforcements, preferably wherein the reinforcements are biodegradable.
46. The prosthesis according to statement 45, wherein the reinforcements are at least at the proximal end of the inner conduit.
47. The prosthesis according to statement 45 or 46, wherein the reinforcements are at least at or near the commissures.
48. The prosthesis according to statements 45 to 47, wherein the reinforcements are bioresorbable rods, such as depicted in Figure 8.
49. The prosthesis according to any of statements 1 to 26 and 39 to 48, wherein the outer conduit and if present the T-shaped conduit biodegrades faster than the inner conduit.

The appended claims are hereby also explicitly incorporated by reference.

### Figure legends

The following numbering refers to: (100) vascular valved prosthesis, (200) inner conduit, (250) focal commissure - folded inner conduit, (300) outer conduit, (330) attachment means - between the inner and outer conduits, (350) protrusions - situated on the outer conduit, (360) focal connection points - between the inner and outer conduits, (400) T-shaped conduit, (430) attachment means - between the outer and T-shaped conduits, (450) bifurcation - situated on T-shaped conduit, (500) distal increased thickness of the inner conduit, (510) inner reinforcement ring, (520) outer reinforcement ring, (600) mandrel, (620) (cylindrical) mandrel core, (640) shell piece(s), (660) shell pieces fixation means, (650) protrusion - situated on mandrel, (670) electrospinning set-up connector means, (700) bifurcated mandrel, (730) mandrel trunk - forming the connection to the outer conduit, (720) bifurcated mandrel core, (740) bifurcated shell pieces, (750) bifurcation - situated on bifurcated mandrel, (760) shell pieces fixation means, (770) electrospinning set-up connector means.

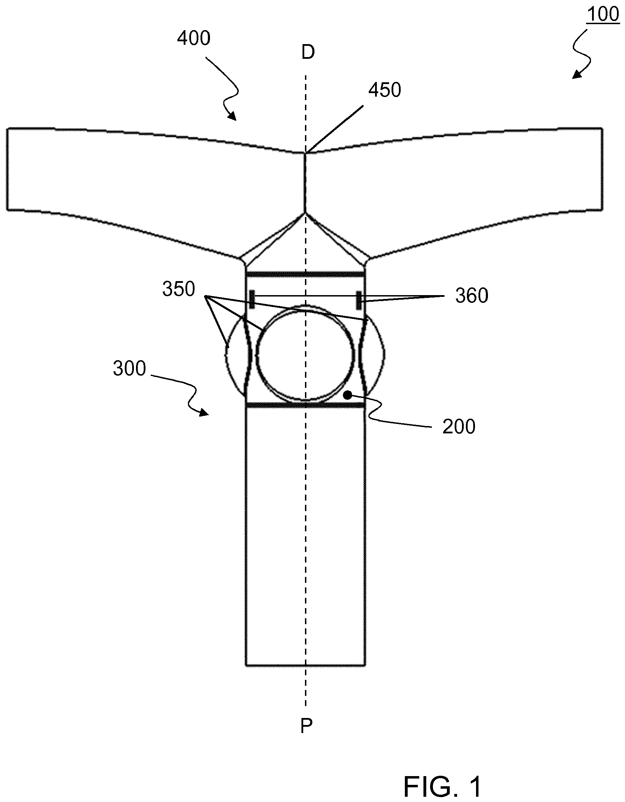

**FIG. 1****:** Schematic cross-section of a vascular valved prosthesis (100) according to an embodiment of the present invention; the vascular valved prosthesis (100) comprises an inner conduit (200) disposed within an outer conduit (300) that is connected to a T-shaped conduit (400). In this embodiment, the inner conduit (200) is configured to function as a valve in a way that fluidic flow may be enabled from a proximal (P) end to a distal (D) end, but may be restricted from D to P; thus allowing unidirectional flow of a fluid through said vascular valved prosthesis (100). The T-shaped conduit (400) and the protrusions (350) are optional.
**FIG. 2****:** Schematic illustration according to an embodiment of an electrospun vascular valved prosthesis (100) according to the present invention. The T-shaped conduit (400) and the protrusions (350) are optional.
**FIG. 3****:** Schematic opened view according to an embodiment of an electrospun vascular valved prosthesis (100) with an open inner conduit (200), according to the present invention. The T-shaped conduit (400) and the protrusions (350) are optional.
**FIG. 4****:** Schematic opened view according to an embodiment of an electrospun vascular valved prosthesis (100) with a closed inner conduit (200), according to the present invention. The T-shaped conduit (400) and the protrusions (350) are optional.
**FIG. 5****:** Schematic illustration according to an embodiment of a mandrel (600) for electrospinning the inner conduit (200), according to the present invention.
**FIG. 6****:** Schematic illustration shows an embodiment of a mandrel (600) for electrospinning the outer conduit (300), according to the present invention.
**FIG. 7****:** Schematic illustration shows an embodiment of a bifurcated mandrel (700) for electrospinning the T-shaped conduit (400), according to the present invention.
**FIG. 8****:** Schematic illustration of an electrospun inner conduit (200) according to an embodiment of the present invention.
**FIG. 9****:** Depicts in an embodiment an electrospun conduit manufactured with a collapsible mandrel according to an embodiment of the invention (b) compared to an electrospun prosthesis manufactured with a non-collapsible, monolithic mandrel according to a prior art method (a).

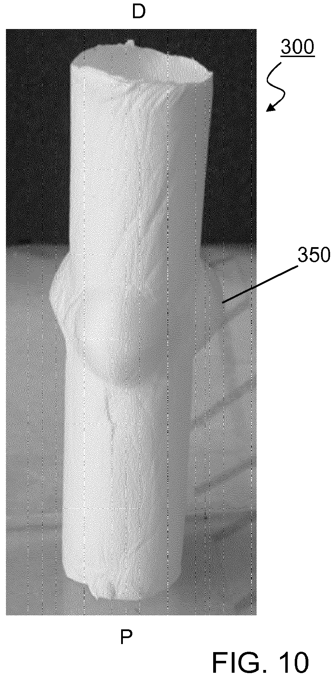

**FIG. 10****:** Depicts an embodiment of electrospun outer conduit (200) manufactured with a collapsible mandrel (600) according to the present invention.
**FIG. 11****:** Demonstrates an embodiment of a front view of a tubular, vascular valved prosthesis (100) according to the present invention.
**FIG. 12****:** Demonstrates an embodiment of a top view of a tubular, vascular valved prosthesis (100) according to the present invention.
**FIG. 13****:** Demonstrates an embodiment of a front view of a T-shaped, vascular valved prosthesis (100) according to the present invention.
**FIG. 14****:** Demonstrates an embodiment of a side view of a T-shaped, vascular valved prosthesis (100) according to the present invention.
**FIG. 15****:** Demonstrates an embodiment of a bottom view of a T-shaped, vascular valved prosthesis (100) according to the present invention.
**FIG. 16****:** Demonstrates an embodiment of an assembly of a mandrel (600) for electrospinning the inner conduit (200).
**FIG. 17****:** Demonstrates an embodiment of an assembly of a mandrel (600) for electrospinning the outer conduit (300).
**FIG. 18****:** Demonstrates an embodiment of an assembly of a bifurcated mandrel (700) for electrospinning the T-shaped conduit (400).

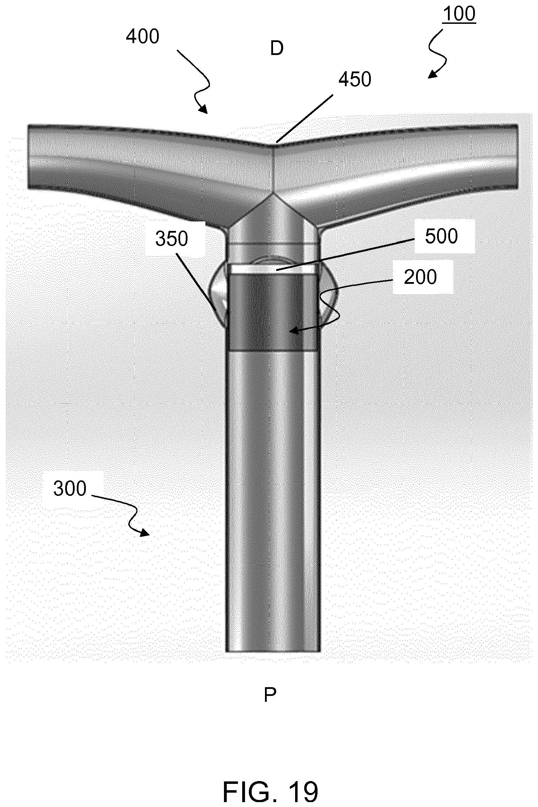

**FIG. 19****:** Schematic illustration according to an embodiment of an electrospun vascular valved prosthesis (100) according to the present invention having a distal over-thickness (500) of the inner conduit. The T-shaped conduit (400) and the protrusions (350) are optional.
**FIG. 20****: A.** Schematic illustration according to an embodiment of an electrospun vascular valved prosthesis (100) according to the present invention having an inner reinforcement ring (510). B. Demonstrates an embodiment of a bottom view of a T-shaped, vascular valved prosthesis (100) according to the present invention having an inner reinforcement ring sutured inside the outer conduit below the sinuses. The T-shaped conduit (400) and the protrusions (350) are optional.
**FIG. 21****:** Schematic illustration according to an embodiment of an electrospun vascular valved prosthesis (100) according to the present invention having an outer reinforcement ring (520). The T-shaped conduit (400) and the protrusions (350) are optional.
**FIG. 22****:** Suture design of the inner conduit to the outer conduit according to various embodiments according to the invention. **A.** Curved vertical suture. **B.** Hill shaped suture. C. Hill shaped + vertical suture. **D.** short vertical suture (5 mm).

### Detailed description

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members. All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The term "vascular valved prosthesis" as used herein refers to a structure suitable for bypassing or replacing a blood vessel section containing a weakened or malfunctioning valve. In the current invention it comprises an inner conduit and an outer conduit, in which the inner conduit is configured as a valve to allow unidirectional flow of a fluid through the outer conduit. From here on forward the terms "vascular valved prosthesis" and "prosthesis" may be used interchangeably and should be regarded as synonymous.

In some embodiments the prosthesis may further comprise additional structures or components, such as a bifurcation, reinforcements, commissures, protrusions, or other forms adding functionality to said prosthesis.

The term "mandrel" as used herein refers to a structure comprising multiple components, such as a mandrel core, fixation mean and shell pieces. Preferably the mandrel is used as a scaffold during electrospinning for the production of prosthesis.

In some embodiments a mandrel may further comprise additional structures or components, such as shell pieces, trunks, commissures, protrusions, or other forms which add functionality to said mandrel and consequentially also to a prosthesis manufactured using said mandrel.

The term "electrospinning" as used herein refers to an electrostatic fiber fabrication technique method which uses electric force to stack charged fibers, fiber solutions or fiber melts, around or within a shape of choice. The fiber diameter is typically in the order of micrometers, but diameters even thinner or thicker are also possible, depending on the resolution and limitations of the setup.

The standard laboratory setup for a person skilled in the art to practise electrospinning typically comprises a (1) spinneret, i.e., a device used to extrude a solution or melt, for example a hypodermic syringe needle, which is connected to a (2) power supply, typically providing five to fifty kV of direct current, in addition to (3) a syringe pump, configured to pump the solution or melt to the spinneret, and (4) a grounded collector, which gathers the extruded solution or melt, for example a spinning wheel or a mandrel.

In some embodiments the electrospinning setup is provided with fibers, preferably in the micro- or nanometer range, or with gels or melts. In further embodiments biologically active compounds may be comprised within the provided materials, preferably selected from peptides, growth factors, biological hydrogels or stem cells. Said biologically active compounds may further comprise functional tissues showing a level of biodegradability.

The term "diameter" as used herein refers to the maximal distance between two antipodal points of an object, i.e., diametrically opposite points lying on the edge of for instance the prosthesis inner wall, running as a straight line segment while passing through the center of the prosthesis. For the present invention the diameter is expressed in units of meter, preferably millimeter (mm). The term "length" as used herein refers to the most extended dimension of an object, defined as the maximal distance between two most extended points of said object. For the present invention the length is expressed in units of meter, preferably millimeter (mm).

The term "wall thickness" as used herein refers to the thickness of the wall that partly seals the inside of said object from the outside, defined as half the difference between the outer diameter and the inner diameter of said object. For the present invention the length is expressed in units of meter, preferably millimeter (mm).

The term "elastic regime" as used herein refers to the ability of the prosthesis to resist a distorting influence or stress, i.e., an external force per unit area, and to return to its original size and shape when the stress is removed. For the present invention the elastic regimen is expressed in terms of percentage (%), wherein zero percent is defined as the prosthesis permanently distorting as a result of any force high enough to cause a distortion, and a hundred percent is defined as the prosthesis resisting any deformation as a result of any force which does not exceed the material strength in magnitude. The elastic regimen is measured with a tensile testing machine.

The term "Young's modulus" as used herein refers the ratio of the stress, i.e., force per unit area, along an axis to the strain, i.e., proportional ratio of deformation over initial length, along that axis in the range of stress in which Hooke's law holds. The young's modulus in the field of the present invention may be determined in the circumferential direction, i.e., deformation of the diameter of the prosthesis 'around' the symmetry axis; and in the radial direction, i.e., deformation of the length of the prosthesis 'out' of the symmetry axis. For the present invention the Young's modulus is expressed in units of Pascal, preferably MegaPascal (MPa), and is measured with a tensile testing machine.

The term "Lagrangian strain" as used herein refers the extensibility of a prosthesis, defined as the finite, relative change in dimension, i.e., deformation, relative to the original, reference dimension resulting from a distorting influence or strain. The Lagrangian strain in the field of the present invention may be determined in the circumferential direction, i.e., deformation of the diameter of the prosthesis 'around' the symmetry axis; and in the radial direction, i.e., deformation of the length of the prosthesis 'out' of the symmetry axis. For the present invention the Lagrangian strain is expressed in units of Pascal, preferably MegaPascal (MPa).

The term "swelling ratio" as used herein refers to the extent of swelling of the prosthesis, defined as the fractional increase in the dimension or volume of the prosthesis due to fluidic absorption. For the present invention the swelling ratio is expressed in percentage (%).

The term "pore size" as used herein refers to the mean diameter of an opening in a surface of an object, through which gases, liquids, or microscopic particles may pass. More specifically, it is an estimate or index of the ratio of the void within a material to the total volume occupied by the material including the voids (cfr. ANSI 7198). For the present invention the pore size may be expressed by a "pore size range" in units of meter, preferably millimeter (mm).

The term "porosity" as used herein refers to the fraction of void (empty) spaces in terms of volume in an object over the total volume of said object. For the present invention the porosity is expressed in percentage (%).

The term "fiber orientation" as used herein refers to the alignment of material in an object relative to the object; concretely, for the present invention the fiber orientation refers to the direction of fiber deposition during electrospinning. When the fiber orientation of an object is found to display a level of anisotropy, i.e., directional dependency, the fiber orientation may be expressed by using an "anisotropic ratio", which is defined as the ratio of anisotropic fiber orientation to isotropic fiber orientation.

In a first aspect of the invention, the invention comprises a biodegradable electrospun vascular valved prosthesis.

The terms "degradability" or "biodegradability" as used herein refer to the complete dissolution of the prosthetic material. Consequently, the "biodegradation rate" corresponds to a period of time; namely, the time for which it takes for the prosthetic material to dissolve and lose at least a part of its mechanical properties. In particular, the biodegradable vascular valved prosthesis according to the invention comprises an electrospun inner conduit having a proximal end and a distal end, and which is disposed within an electrospun outer conduit having a proximal end and a distal end, wherein the inner conduit is attached to the outer conduit such as to function as a valve allowing unidirectional flow of a fluid through said outer conduit from the outer conduit's proximal to distal end.

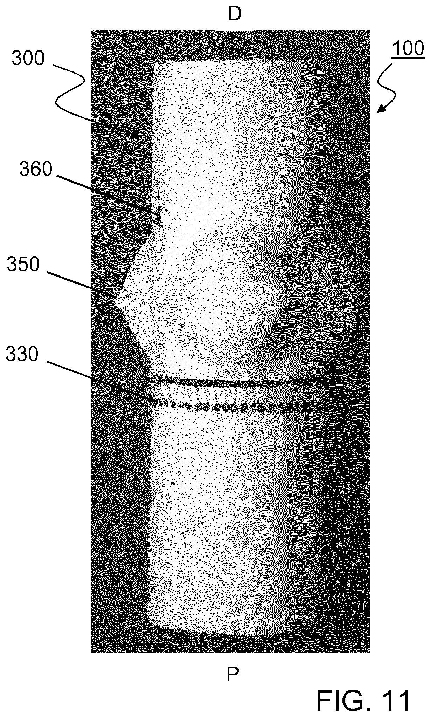

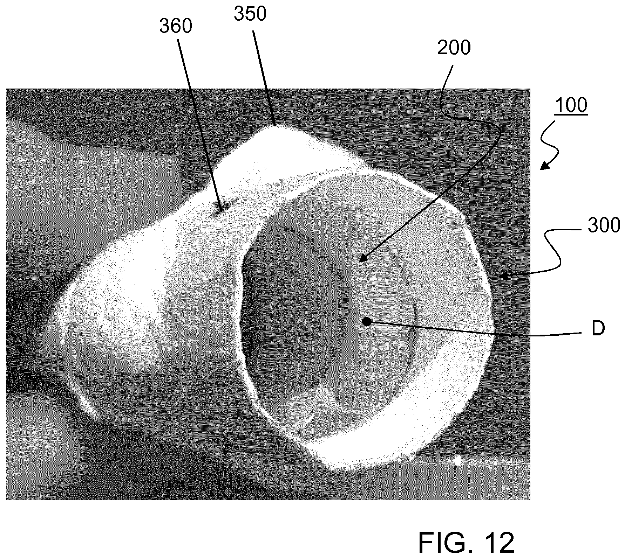

According to some embodiments an electrospun inner conduit disposed within an electrospun outer conduit has a proximal (P) end and a distal (D) end, wherein the inner conduit is attached to the outer conduit and is configured to enable unidirectional flow of a fluid through said outer conduit from the outer conduit's P to D end, such as to function as a valve. Preferably an inner conduit is configured to allow unidirectional flow of a fluid once it is comprised within the structure of an outer conduit. A demonstration of an inner conduit disposed within an outer conduit may be observed in figures 11 and 12; specifically, figure 11 provides a frontal view of the outside, while figure 12 a top view from the inside.

The entry point through which the fluid is supplied to the prosthesis is hereby referred to as the "entry", and the exit point through which the fluid leaves the prosthesis is hereby referred to as the "exit". Concretely, the P end corresponds to the direction through which a fluid preferably may be provided into an outer conduit, but once passed through the inner conduit would be unable to return through the same entry; whereas the D end corresponds to the direction through which said fluid that preferably may have been provided would be able to exit the outer conduit; therefore a flow of fluid is enabled from a P to D, but is restricted from D to P, such as to function as a valve. Accordingly, figure 12 thus shows a demonstration of the exit from the D end.

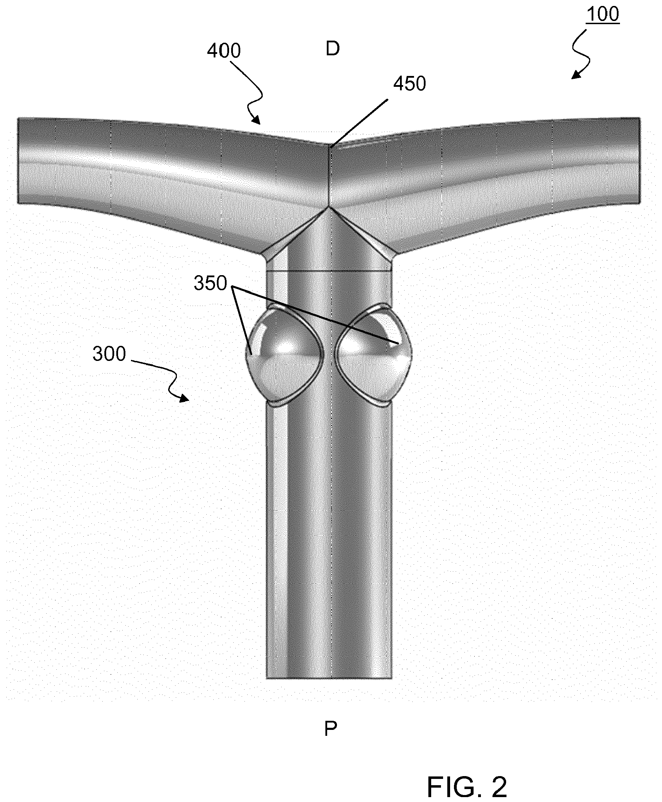

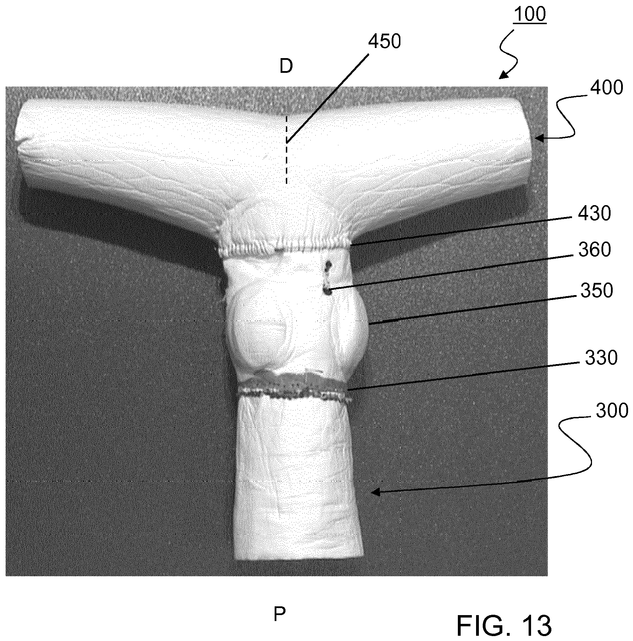

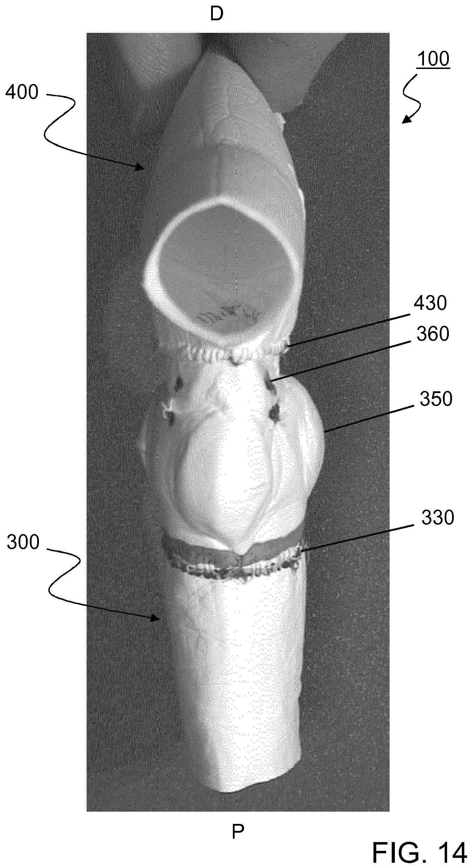

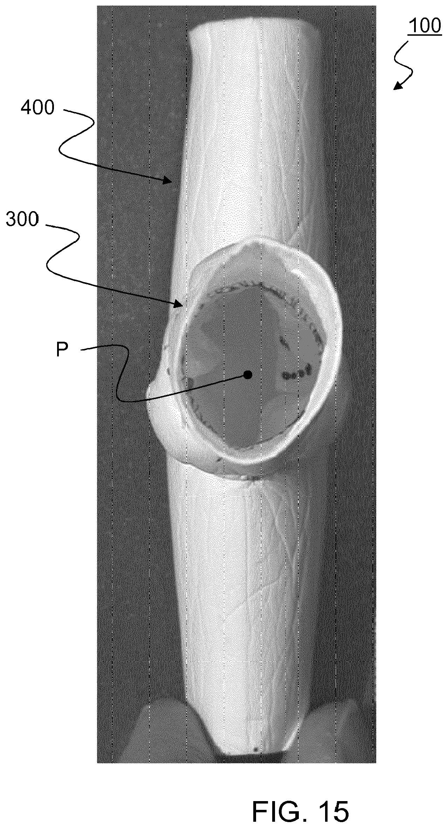

A schematic cross-section according to an embodiment may be found in figure 1 which shows a vascular valved prosthesis (100) with an inner conduit (200) disposed within an outer conduit (300). The proximal (P) and distal (D) ends show the flow direction through this embodiment of the prosthesis. Figure 2 provides a graphical model of the same embodiment. Figures 13 to 15 further demonstrate an exemplary assembly of the same embodiment; figure 13 provides a view from the front, figure 14 from the side and figure 15 from the bottom.

In certain embodiments, the outer conduit has different properties than the inner conduit. In certain embodiments, the outer conduit has different mechanical properties than the inner conduit. In certain embodiments, the outer conduit has a different biodegradation rate than the inner conduit.

It will be understood that when referring to the outer conduit, in particular certain properties of the outer conduit, the same may apply to the T-shaped conduit, if and when present in the prosthesis according to the invention. For instance, when referring to biodegradability of the outer conduit, in certain preferred embodiments, the biodegradability of the T-shaped conduit is the same as the biodegradability of the outer conduit.

By using a combination of two conduits there are benefits for production and structural functionality of a prosthesis. First, by allowing separate production times for both conduits the total production time for the prosthesis may be decreased for both through simultaneous production. Second, it may allow a higher degree of electrospinning control during production, especially when using biologically active compounds. Third, a post-production inspection for each conduit may be easier to perform. Fourth, a level of production flexibility may be obtained by allowing the shapes and materials of the inner conduit, i.e., valves and the outer conduit, i.e., the tubular prosthesis, to be designed separately. Therefore, if a patient pathology would require specific, on-demand adjustments to either one of the conduits, these could be produced without adjusted the entire production mechanism. These adjustments may, for example, include different shapes, lengths, diameters, or even different biologically active compounds; in case a patient has a certain profile which could incite nefarious effects, such as unwanted biological deposits, allergic reactions, etc. Fifth, the use of a tube-like conduit to create a valve inside another tubular conduit is highly efficient in term of functionality (i.e., competence and hemodynamic) of the valve.

In some embodiments an inner conduit is smaller in diameter than an outer conduit. In some embodiments, an inner conduit has the same or substantially the same diameter than an outer conduit, e.g., the outer diameter of the inner conduit (i.e. the lumen diameter plus twice the wall thickness) is the same or substantially the same as the inner diameter (i.e. the lumen diameter, preferably excluding any eventually present protrusions) of the outer conduit. Such embodiments allow the inner conduit to be comprised within the structure of an outer conduit; more preferably, the diameter may be sufficiently large to prevent any leakage which would impair the valve functionality of the inner conduit. In some embodiments an inner conduit is larger in diameter than an outer conduit; such embodiments may be beneficial to improve the closing of the valve.

In some embodiments the prosthesis may be bifurcated; preferably the bifurcation is T-shaped. By incorporating a T-shaped bifurcation directly into the structure of the prosthesis, the prosthesis may better mimic the natural shape of a branching artery, and will allow better functionality as well as better structural stability and integrity.

In preferred embodiments, the bifurcation is located towards the distal end of the outer conduit, so as to split the volume fluid after passing through the inner conduit. An exemplary bifurcation may be found in figure 1, which shows a vascular valved prosthesis (100) comprising an outer conduit (300) that has been attached to a T-shaped conduit (400), i.e. to the trunk of the T-shaped conduit, which comprises said bifurcation (450). As can be seen from the figure, the bifurcation is located at the distal (D) end of the prosthesis (100). Figure 2 provides a graphical model of the same embodiment. Figures 13 to 15 further demonstrate an exemplary assembly of the same embodiment; specifically, figure 13 provides a view from the front, in figure 14 from the side and in figure 15 from the bottom.

In some embodiments the prosthesis may replace a vessel section comprising a pulmonary valve or an aortic valve.

The advantage of a bifurcation, which is preferably T-shaped, is that it may allow a better compatibility with arterial systems showing a branching situated very close to a faulty valve. Examples of such arterial systems are those surrounding a pulmonary or aortic valve, but also other vascular regions or structures may benefit from the bifurcation. Prosthesis replacement of these arterial systems using a single tubular prosthesis would not provide enough fluidic flow to all branches; and would thus be an inadequate solution for certain patient pathologies. Moreover, these bifurcation branches are also biodegradable and may lead to a complete regeneration by autologous tissue. Thus, such autologous branches will have a growth potential, which is particularly important in children. Additionally, it may decrease the difficulty of surgically connecting one prosthesis to several branches, or different prostheses together.

In some embodiments the outer conduit comprises at least one radially outward protrusion.

In some embodiments an outer conduit, and by extension the prosthesis, comprises at least one radially outward protrusion. In certain embodiments, such protrusion(s) may advantageously mimic (and hence replace) naturally occurring sinuses. These protrusions advantageously aid in the adequate mechanical functioning of the valve. Their presence may create a "Venturi effect", i.e., a reduction in fluid pressure when a fluid flows through a constricted section of pipe, which may help achieve a complete opening and closing of the valve with the lowest possible amount of shear stress and fatigue. Additionally, they may also reduce the time required for the opening and closing of the valve.

In some embodiments the prosthesis may be shaped to resemble natural arterial protrusion, such as to include a sinus, cavity, sacks, or other similarly shaped features. By implementing protrusions in an outer conduit different fluid rates may be obtained to achieve different valve actions. Resulting from the implementation of one or more protrusions the prosthesis may better resemble the functionality of a natural artery, and may improve fluid pressure and flow control which may prove beneficial for patient response and recovery rate.

In some embodiments the proximal end of said inner conduit is attached circumferentially along the inside of the outer conduit towards the proximal end of the outer conduit to form a circumferential commissure; wherein the distal end of said inner conduit is attached to the inside of the outer conduit towards the distal end of said outer conduit to form focal commissures at one or more discrete positions, preferably two or more discrete positions, such as three discrete positions, wherein the focal commissures may or may not extend longitudinally from the distal end of the inner conduit towards the proximal end of the inner conduit. The vertical commissures may help achieve the "valve-like" functionality of the inner conduit, and thus of the prosthesis. Preferably, the focal commissures are equidistantly spaced. Preferably, the focal commissures are coplanar.

In some embodiments the inner conduit may exhibit a vertical commissure situated along the wall of the inner conduit; preferably, two or more symmetrical commissures may be present.

The inclusion of commissures may allow the inner conduit to expand or contract in diameter outside of the range determined by the material elasticity. This may enable easier assembly of the inner conduit within the outer conduit.

Additionally, the vertical commissures may improve valve dynamics and fatigue life during operation of the inner conduit.

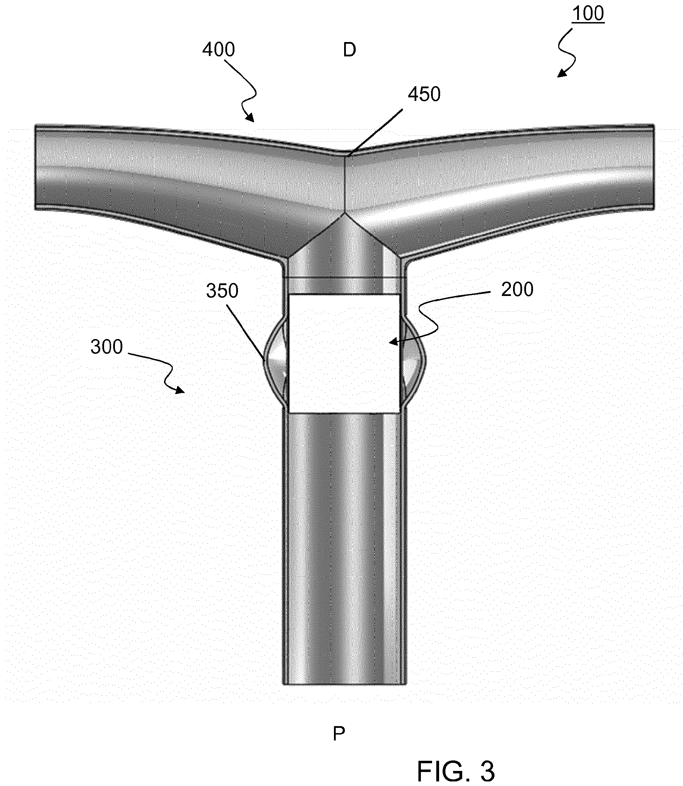

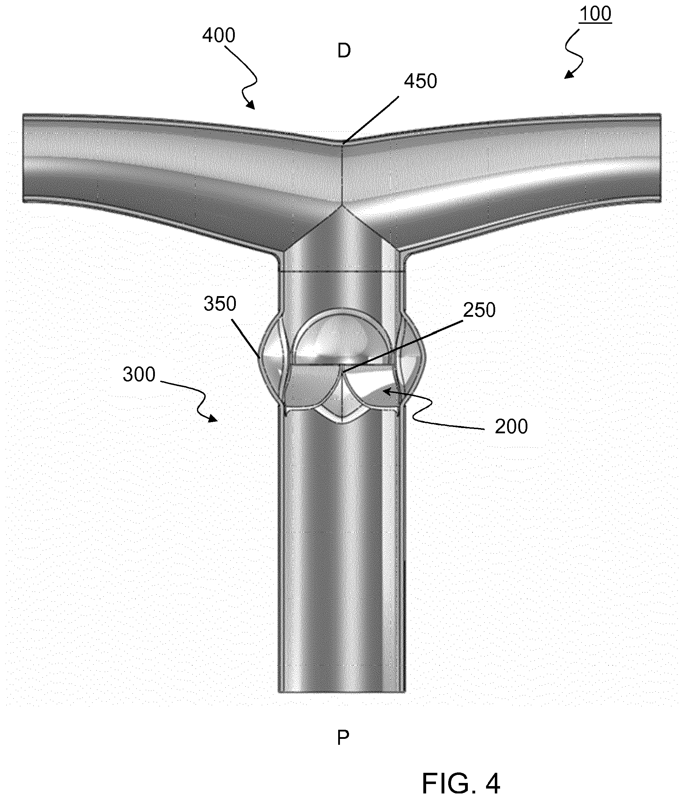

To better clarify this technical feature, figures 3 and 4 demonstrate a prosthesis (100) with an open inner conduit (200) and a closed inner conduit (200), respectively. In figure 3 the liquid may flow freely through the prosthesis (100); however, in figure 4 the focal commissures (250) will allow flow of a liquid from the proximal (P) end to the distal (D) end prevent, but prevent any flow from the D to the P. As such, a valve-like functionality is obtained for the prosthesis resembling that of a sinus of Valsalva, i.e., an aortic sinus. During heartbeat the prosthesis can thus be envisioned as an aorta or a pulmonary artery wherein the inner conduit functions as sinus of Valsalva; so that during systole, cfr. figure 3, the inner conduit may allow the circulation of a fluid through and during diastole, cfr. figure 4, the inner conduit may prevent the backflow of the passed fluid. Additionally, it may be noted that in both figures the T-shaped conduit is fixed to the outer conduit in the direction of the circulating fluid, similar to pulmonary artery or aortic branches.

In some embodiments the inner conduit may be set up to the outer conduit with the use of circumferential cohesion lines; said cohesion lines are configured in a way to allow a proper positioning of the inner conduit with regard to the outer conduit. The advantage of using cohesion lines is that it may allow a better positioning and placement of an inner conduit within an outer conduit; thus forming a vascular valved prosthesis. A proper placement may allow a high degree of competence and functionality, and may in turn reduce the risks of prosthesis malfunctioning, such as leakage or obstruction.

In some embodiments the distal end of the inner conduit can exhibit a thickness characterized by a larger wall width, situated in the direction of the center of the outer conduit. The addition of a thickness at said location may improve the closing of the inner conduit after fluid is pumped through said inner conduit, for example, during diastole.

In some embodiments the outer conduit comprises three coplanar radially equidistally spaced outward protrusions (e.g. sinus-like structures), and wherein the distal end of said inner conduit is attached at three equidistally spaced discrete positions to the inside of the outer conduit along longitudinal lines separating the protrusions.

In some embodiments the inner and/or outer conduit may further exhibit a longitudinal commissures situated along the wall of the inner conduit; preferably, at least two symmetrical commissures may be present; most preferably, three equidistally spaced commissures may be present. Such additional commissures function to further strengthen the prosthesis (cfr. figure 8). In some embodiments the distal end of the inner conduit is attached to the inside of the outer conduit towards the distal end of said outer conduit to form focal commissures at one or more discrete positions, preferably two or more discrete positions, such as three discrete positions, wherein the focal commissures may extend longitudinally from the distal end of the inner conduit towards the proximal end of the inner conduit. Preferably, the focal commissures are equidistantly spaced. Preferably, the focal commissures are coplanar.

In some embodiments a T-shaped conduit is attached along the distal end of said outer conduit.

In some embodiments the diameter of the proximal end (i.e. the trunk) of the T-shaped conduit could be slightly reduced vis-à-vis the diameter of the outer conduit (or vice versa), in order to fit inside the outer conduit (or vice versa) to facilitate its fixation with the outer conduit with protrusions.

In some embodiments the prosthesis comprises biologically active compounds.

Preferably, the biologically active compound is selected from peptides, growth factors, biological hydrogels such as gelatin, and/or may comprise stem cells such as adipose-derived stem cells, and/or combinations thereof.

Biologically active compounds may promote an improved level of (exogenous) cell seeding and tissue regeneration beneficial for the recovery rate of a patient. Seeding of stem cells may increase tissue regeneration speed ultimately replacing the prosthesis with newly grown tissue for which the prosthesis may serve as a scaffold. Alternatively, tissue regeneration may entirely or at least partially rely on cells already present in the patient's body by using for instance the biomaterial of the prosthesis as a signalling mechanism for driving endogenous cells towards the scaffold; possibly after appropriate stimulation of cell expansion and/or differentiation.

In some embodiments the inner conduit is circumferentially or longitudinally reinforced by biodegradable structures. In some embodiments the outer conduit is circumferentially or longitudinally reinforced by biodegradable structures. In some embodiments the prosthesis is circumferentially and longitudinally reinforced by biodegradable structures.

In some embodiments, the outer or inner conduit is circumferentially or longitudinally reinforced by additional biodegradable structures, optionally wherein the additional biodegradable structures form a ring which is disposed on the inside or on the outside of a part of the conduit. The reinforcements may be made from the same or different material as the inner or outer tube. In certain embodiments, the reinforcements are electrospun.

In certain embodiments, longitudinal reinforcements extend along the entire length or substantially the entire length of the outer or inner conduit. In certain embodiments, longitudinal reinforcements extend along part of the length of the outer or inner conduit. In certain embodiments, reinforcements, such as longitudinal or circumferential reinforcements, are provided at least at or near the commissures. In certain embodiments, reinforcements, such as longitudinal or circumferential reinforcements, are provided on the inner conduit at least at the proximal end.

Such reinforcements, and in particular the reinforcement ring, can minimize the flexure when the inner conduit is actively opening and closing. Accordingly, the functionality of the inner conduit over time is improved. If reinforcements are provided at or near commissures, external forces applied on the commissures can be decreased.

It will be understood that the reinforcements may be attached to the conduits by means described herein elsewhere, such as including by suture, stapling, gluing, welding (laser, vibration, ultrasonic, induction, high frequency) or a combination of the processes described. Alternatively, the reinforcements may be added during manufacturing of the conduit, for instance by electrospinning.

In certain embodiments, the reinforcements are local areas of the conduit having a thicker conduit wall. Accordingly, in certain embodiments, the outer or inner conduit comprises circumferential or longitudinal sections or areas, such as a ring, which sections or areas have an increased wall thickness relative to the remainder of the conduit wall.

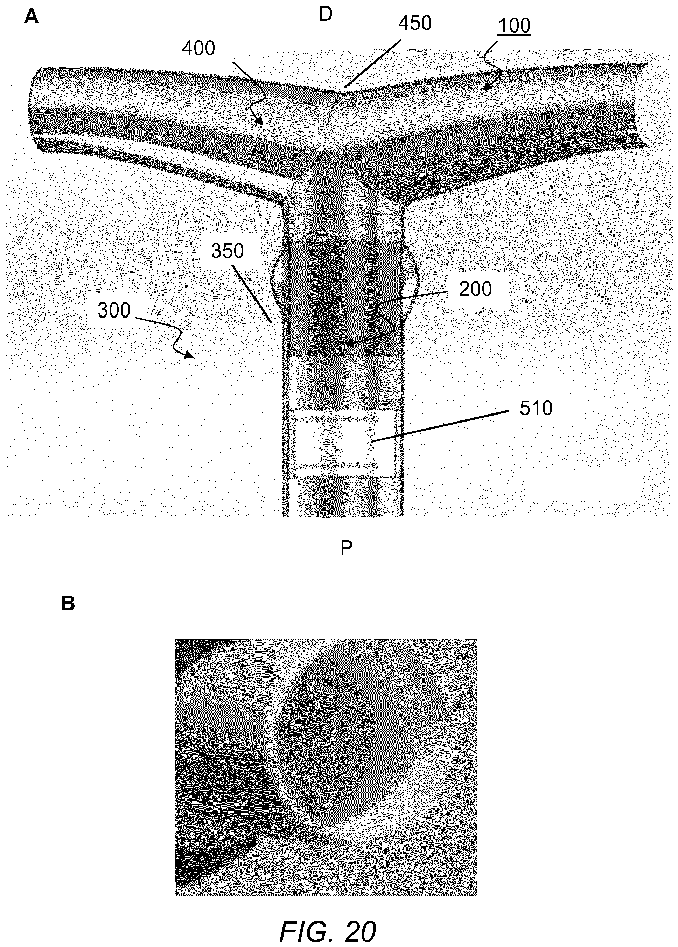

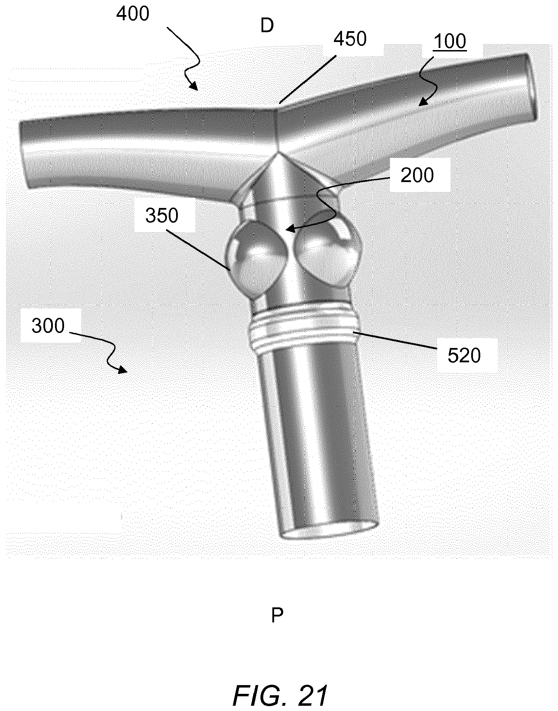

In certain embodiments, the outer conduit comprises one or more circumferential reinforcements, preferably a ring along the inside or outside of the conduit, wherein the reinforcement is located at or near the proximal end of the inner conduit, as for instance illustrated in Figures 20 and 21. In certain embodiments, the outer conduit comprises one or more circumferential reinforcements, preferably a ring along the inside or outside of the conduit, wherein the reinforcement is located at or near the proximal end of the protrusions.

In certain embodiments, the reinforcements increase the wall thickness by at least 10%, such as at least 20%, at least 30%, at least 40%, at least 50%, at least 60%, at least 70%, at least 90%, at least 90%, at least 100%, or more, such as at least 150%, at least 200%, or more. In certain embodiments, the reinforcements do not increase the wall thickness by more than 400%, preferably by no more than 300%.

In certain embodiments, the circumferential reinforcement has a length of between 1% and 50% of the length of the conduit (i.e. the distance between the distal and proximal end of the conduit), such as between 5% and 25%. In certain embodiments, the circumferential reinforcement has a length of between 1 mm and 50 mm, such as between 3 mm and 20 mm, or between 5 mm and 10 mm.

Reinforcements of the prosthesis manufactured using biodegradable structures may over-time naturally decompose; thus allowing the implantation of temporary reinforcements. This could prove beneficial for reinforcements which might otherwise require a secondary invasive procedure for their removal, which might be detrimental to patient recovery rate.

In certain embodiments, the inner conduit at its distal end has a wall thickness which is larger than the wall thickness at the proximal end. Such increased wall thickness at the distal end improves closure of the valve. In certain embodiments, at least the most distal 10% has a wall thickness which is larger than the wall thickness at the proximal end. In certain embodiments, at least the most distal 20% has a wall thickness which is larger than the wall thickness at the proximal end. In certain embodiments, at least the most distal 30% has a wall thickness which is larger than the wall thickness at the proximal end. In certain embodiments, at least the most distal 40% has a wall thickness which is larger than the wall thickness at the proximal end. In certain embodiments, at least the most distal 50% has a wall thickness which is larger than the wall thickness at the proximal end. In certain embodiments, at most the most distal 10% has a wall thickness which is larger than the wall thickness at the proximal end. In certain embodiments, at most the most distal 20% has a wall thickness which is larger than the wall thickness at the proximal end. In certain embodiments, at most the most distal 30% has a wall thickness which is larger than the wall thickness at the proximal end. In certain embodiments, at most the most distal 40% has a wall thickness which is larger than the wall thickness at the proximal end. In certain embodiments, at most the most distal 50% has a wall thickness which is larger than the wall thickness at the proximal end. In certain embodiments, the wall thickness of between 1 mm and 20 mm of the most distal portion of the conduit is larger than the wall thickness of the proximal end, such as between 2 mm and 10 mm, or between 2 mm and 5 mm. In certain embodiments, the the wall thickness is increased by at least 10%, such as at least 20%, at least 30%, at least 40%, at least 50%, at least 60%, at least 70%, at least 90%, at least 90%, at least 100%, or more, such as at least 150%, at least 200%, or more. In certain embodiments, the wall thickness is not increased by more than 400%, preferably by no more than 300%, more preferably by no more than 200%, most preferably by no more than 100%. It will be understood that the section having increased wall thickness may be continuous (i.e. completely circumferential) or may be segmented (e.g. interrupted at the focal commissures).

According to the invention the prosthesis is manufactured using biodegradable compounds. A prosthesis manufactured using biodegradable structures may over-time naturally decompose; thus allowing the implantation of temporary prosthesis. For this purpose the biodegradation rate is determined by the persistence of the mechanical properties, and a structure is fully degraded once the mechanical properties of the structure are gone. As used herein, the terms "biodegradation" and "degradation" refer to the same biochemical process. Biodegradation rate can depend on the materials used (e.g. type of polymer), but can also depend on particular physical properties, such as pore density, thickness of the conduit, etc. In certain embodiments, the degradation rate of the inner conduit can be slower as the degradation rate of the outer conduit. In certain embodiments, the inner and outer conduit are made of the same materials. Hence, due to the thickness of the inner and outer conduit, the biodegradation rate will be different. Biodegradation could prove beneficial for regenerative strategies where the fault valve may be regenerated using the prosthesis as a temporary mold for cell attachment or deposition; preferably the prosthesis will further promote cell seeding and attachment which may be beneficial for patient recovery rates and long-term health. In certain embodiments, the entire prosthesis is biodegradable, i.e. the prosthesis is fully biodegradable. This biodegradability is at the very basis of the regeneration of the prosthesis, potential for growth. The main advantages of this approach is to avoid re-interventions to surgically replace the outgrown prosthesis, reduce risk of surgical complications (e.g., bleeding, infection, heart block, stroke, renal failure, death) and minimise the human, social and financial costs related to these re-interventions and complications. This feature is especially important for prosthesis implanted in children, which may outgrow the prosthesis.

In some embodiments the inner conduit when implanted in a vascular structure degrades (i.e., biodegradation) in a period ranging from at least 6 months to at most 48 months; preferably between 12 to 36 months; most preferably between 18 to 30 months; most preferably in about 24 months. In some embodiments the outer conduit when implanted in a vascular structure degrades in a period ranging from at least 1 month to at most 24 months; preferably between 3 to 18 months; most preferably between 6 to 12 months. In some embodiments the T-shaped conduit when implanted in a vascular structure degrades in a period ranging from at least 1 month to at most 24 months; preferably between 3 to 18 months; most preferably between 6 to 12 months. In preferred embodiments the outer and T-shaped conduits have essentially the same degradation rate. In some embodiments the biodegradability of the inner conduit is different from the biodegradability of the outer conduit. In some embodiments the biodegradability of the inner conduit is essentially the same as the biodegradability of the outer conduit.

In preferred embodiments the biodegradability of the inner conduit is slower than biodegradability of the outer conduit; preferably, the biodegradability is two times as slow; more preferably the biodegradability is three times as slow. This slower biodegradability of the inner conduit guarantees the persistence of the competence of the valved part of the device until the newly regenerated tissue displays good mechanical properties.

By implementing different biodegradability rates along the diameter of the prosthesis, the rate of tissue regeneration may be promoted from the outside conduit towards the inside conduit.

In a preferred embodiment the rate of biodegradability may be adjusted to a patient pathology and regeneration rates, for example, young patients may benefit from faster prosthesis degradation in comparison to older patients.

In some embodiments the biodegradability of the distal end of the commissures is different from the biodegradability of the proximal end of the commissures. In preferred embodiments the biodegradability of the distal end of the commissures is faster than the biodegradability of the proximal end of the commissures; preferably, the biodegradability is twice as fast; more preferably, biodegradability is thrice as fast. The faster biodegradability of the distal end of the commissures can enhance with time the length of the coaptation surface of the different parts of the inner conduit during the diastole. By implementing different biodegradability rates along the length of the prosthesis, the rate of tissue regeneration may be promoted from the distal end to the proximal end.

In some embodiments the inner conduit is multilayered. In some embodiments the outer conduit is multilayered. In some embodiments both the inner and outer conduits are multilayered. By applying multiple layers over the inner or the outer conduit, material and structural properties of the prosthesis may be enhanced, and each layer may have its own structural and/or functional characteristics (e.g. biodegradability, directional structural reinforcements, fibre orientation, porosity, etc.). Multiple layers may be made from the same or from different materials. For electrospun conduits, the "core" refers the fibers located on the internal part, situated towards the mandrel; while the "shell" refers to the fibers located on the external part, surrounding the core.

The properties of the prosthesis may be enhanced by using layers with different material and structural properties to complement each other; for example, by combining a layer which shows an enhanced biodegradation rate or porosity, yet suffers from a weak material strength, with a layer that has a high material strength, yet has a reduced biodegradation rate or porosity. Thus, by properly designing and combining layers the prosthesis properties may be enhanced in comparison to the properties of singular layers. In some embodiments the layer facing the inside of the prosthesis may have a faster biodegradability rate than the layer facing the outside of the prosthesis. In some embodiments the layer facing the inside of the prosthesis may have a slower biodegradability rate than the layer facing the outside of the prosthesis.

In some embodiments the inner conduit is made by electrospinning of (biodegradable) polymers based on supramolecular chemistry or polymers comprising stereocomplexes. In some embodiments the outer conduit is made by electrospinning of (biodegradable) polymers based on supramolecular chemistry or polymers comprising stereocomplexes. In some embodiments both the inner conduit and said outer conduit are made by electrospinning of (biodegradable) polymers based on supramolecular chemistry or polymers comprising stereocomplexes. In some embodiments the inner conduit is made by electrospinning of (biodegradable) polymers based on supramolecular chemistry or polymers comprising stereocomplexes, wherein the polymers are biodegradable aliphatic polyesters or biodegradable polyurethanes. In some embodiments the outer conduit is made by electrospinning of (biodegradable) polymers based on supramolecular chemistry or polymers comprising stereocomplexes, wherein the polymers are biodegradable aliphatic polyesters or biodegradable polyurethanes. In some embodiments both the inner conduit and said outer conduit are made by electrospinning of (biodegradable) polymers based on supramolecular chemistry or polymers comprising stereocomplexes, wherein the polymers are biodegradable aliphatic polyesters or biodegradable polyurethanes. In some embodiments the inner conduit is made by electrospinning of biodegradable aliphatic polyesters or biodegradable polyurethanes. In some embodiments the outer conduit is made by electrospinning of biodegradable aliphatic polyesters or biodegradable polyurethanes. In some embodiments both the inner conduit and said outer conduit are made by electrospinning of biodegradable aliphatic polyesters or biodegradable polyurethanes. In some embodiments the conduits are made by electrospinning a mixture of (co)polymers, such as biodegradable aliphatic polyesters and biodegradable polyurethanes. In some embodiments the outer conduit is made by electrospinning a combination of biodegradable aliphatic polyesters and biodegradable polyurethanes. In some embodiments both the inner conduit and outer conduit are made by co-electrospinning of (biodegradable) polymers into core-shell fibres, wherein the polymers are biodegradable aliphatic polyesters or biodegradable polyurethanes. In some embodiments both the inner conduit and said outer conduit are made by electrospinning a combination of biodegradable aliphatic polyesters and biodegradable polyurethanes.

The advantages of polyester and polyurethanes are that they may be readily available and can be easily processed through electrospinning. Applied polyester may be extremely versatile in its structural properties, ranging from very soft to very firm and durable. As prosthesis material polyester absorbs less than one percent of moisture by weight, offers good dimensional stability under a variety of conditions, and has unique filling properties. Once implanted, polyester doesn't promote the growth of bacteria making it very suitable for biological applications. Alternatively, applied polyurethane may be extremely versatile in its structural properties, having high tensile and load baring capacities. As prosthesis material polyester will remain stable under fluidic conditions with minimal swelling. Currently, polyurethane (PU) is the polymer of choice in cardiac applications due to its high biocompatibility, strong in vivo performance and simplicity of manufacturing. Artificial heart valves made from polyurethane have high biocompatibilities because of the extensive degrees of freedom that arise from having three component monomer parts (as compared to other polymers, where fewer components are usually employed). This allows for unlimited variation in mechanical and physicochemical characteristics. The advantage of polylactic acid is that it may be readily available from renewable resources, is relatively inexpensive, and easily processed through electrospinning. Additionally, applied polyester may be extremely versatile in its structural properties, ranging from very soft to very firm and durable. As a biocompatible material polylactic acid is neither toxic nor carcinogenic to the human body, hence making it an excellent material for biomedical applications. As a biodegradable prosthesis material polylactic acid may break down inside the body within 6 months to 2 years. This gradual degradation may be desirable for a support structure, because it can gradually transfer the load to the body as that area heals; thus functioning as a scaffold for regenerating tissue.

In preferred embodiments the inner conduit is made by electrospinning a mixture of (co)polymers from the following list: Poly(ethylene glycol) (PEG), Poly(glycolic acid) (PGA), Poly(lactic acid) (PLA), Poly-4-hyrdoxybutyrate (P4HB), Polycaprolactone (PCL), Poly(glycerol sebacate) (PGS), Poly(ester urea urethane) (PEUU), Polydioxaneone (PDO), Polycarbonate (PCU), poly(carbonate urethane urea) (PCUU), Polyhedral oligomeric silsesquioxanes (POSS), and/or combinations thereof. All these polymers offer several advantages towards the manufactory of a vascular valved prosthesis, namely, they are mechanically compatible with the manufacturing process of electrospinning a trifoliate valve with the targeted mechanical functionality, i.e., a degradation rate and long-term efficient hemodynamic opening and closing of the sinuses; they are biocompatible; they can be processed by electrospinning; they are approved by regulatory commissions; they can be functionalized by multiple peptides depending on the chosen technique of peptide/polymer grafting, for example, peptides RGD and SDF1 may be currently selected; they can be seeded by cells; they can be sterilized under industrial conditions without any structural alteration of or exterior damage to the prosthesis; and last, they are readily available for manufactory. In more preferred embodiments aliphatic diisocyanates, such as lysine diisocyanantes (LDI) and 1,4-diisocyanatobutane (BDI), are used as material of choice above aromatic diisocyanates; since the latter was found to form toxic products, e.g., diisocyanates such as 4,4'methylenediphenyl diisocyanate (MDI) and toluene diisocyanate (TDI) upon degradation and was thus found unsuitable as biodegradable biomaterials. Most aliphatic diisocyanates-based polyurethanes have a Young's modulus and tensile strength of several tens of MPa, and a large breaking strain in the range of 100 - 1000%. As such, the selection of a soft and ideally completely elastic material, i.e., 100% recovery from deformation, based on aliphatic diisocyanates is preferred. The susceptibility of polymers to biodegradation is governed by "soft" segment components which are generally glycols, such as polyethylene glycol (PEG) or polycaprolactone diols (PCL). However, higher proportions of soft segments tend to be correlated with an increased degradation rate. Given that the hard segments are known to be highly thrombogenic due to their high crystallinity and strong hydrogen bonding, alternative compositions were sought after and experimented with, such as synthetic materials with hydrophilic soft segments, the addition of functional groups as well as the coating, impregnation and grafting of techniques to modify surfaces. In particular, polyethylene oxide (PEO) was observed to provide a successful surface coating due to its neutral charge and flexibility; it may be utilised as a possible permanent coating to prevent protein surface adsorption to the prosthesis. After conducting a comprehensive survey of potentially suitable materials with regards to the mechanical properties, biodegradability, safety and compatibility with the electrospinning production process, poly(ester urethane)-urea (PEUU) was found to be preferred over poly(carbonate urethane urea) (PCUU).

In some embodiments the inner conduit is attached to the outer conduit by sutures, staples, glue, welds (laser, vibration, ultrasonic, induction, high frequency) or a combination thereof. In preferred embodiments where the prosthesis has a high rate of biodegradability, the choice of attachment will involve a level of biodegradability too; for example, sutures, glue or staples made from a biodegradable material. By properly attaching the inner conduit to the outer conduit the chance for unwanted effects, such as valve leakage or breakage, may be significantly lower.

The attachment is demonstrated in figures 11 to 15 by means of sutures. The former shows the attachment formed between an inner conduit disposed within an outer conduit; thus forming a tubular vascular valved prosthesis. Specifically, figure 11 shows the attachment from the outside in a frontal view; while figure 12 provides a top view to observe the same attachment from the inside. Next, the combined inner and outer conduit may be affixed in a similar manner to the T-shaped conduit, as is demonstrated in figure 13 from the front, in figure 14 from the side and in figure 15 from the bottom; thus forming a T-shaped vascular valved prosthesis.

Though it may be designed to resemble a natural artery, preferably the prosthesis' structure will contain inbuilt reserves of strength and stability above those of a natural artery to better resist the high workload performed by a pumping heart. In certain cases the prosthesis may remain as a (semi-) permanent part of a vascular system, so its structural integrity and reliability can be affected by long-term effects, such as aging or biological deposits.

In some embodiments the prosthesis is characterized in that the inner conduit has a linear elastic regimen of at least 20%; preferably at least 40%; more preferably at least 50%; most preferably at least 60%. In some embodiments the prosthesis is characterized in that the outer conduit has a linear elastic regimen of at least 5%; most preferably at least 10%. In some embodiments the prosthesis is characterized in that the T-shaped conduit has a linear elastic regimen of at least 5%; most preferably at least 10%.

In some embodiments the prosthesis is characterized in that the inner conduit has a Young's modulus in the circumferential direction ranging from at least 0.01 to at most 200 MPa; preferably ranging from 0.1 to 150 MPa; more preferably ranging from 1 to 125 MPa; most preferably ranging from 5 to 100 MPa. In some embodiments the prosthesis is characterized in that the inner conduit has a Young's modulus in the radial direction ranging from at least 0.01 to at most 200 MPa; preferably ranging from 0.05 to 100 MPa; more preferably ranging from 0.1 to 30 MPa; most preferably ranging from 0.5 to 15 MPa. In some embodiments the prosthesis is characterized in that the outer conduit in the radial direction and/or the circumferential direction has a Young's modulus ranging from at least 0.001 to at most 100 MPa; preferably ranging from 0.005 to 10 MPa; more preferably ranging from 0.01 to 1.5 MPa; most preferably ranging from 0.01 to 0.5 MPa. In some embodiments the prosthesis is characterized in that the T-shaped conduit in the radial direction and/or the circumferential direction has a Young's modulus ranging from at least 0.001 to at most 100 MPa; preferably ranging from 0.005 to 10 MPa; more preferably ranging from 0.01 to 1.5 MPa; most preferably ranging from 0.01 to 0.5 MPa. In preferred embodiments the outer conduit and the T-shaped conduit have the same Young's modulus in the circumferential and radial direction.

In some embodiments the prosthesis is characterized in that the inner conduit has a Lagrangian strain in the circumferential direction ranging from at least 0.01 to at most 50 MPa; preferably ranging from 0.04 to 10.0 MPa; more preferably ranging from 0.08 to 1.0 MPa; most preferably ranging from 0.1 to 0.4 MPa. In some embodiments the prosthesis is characterized in that the inner conduit has a Lagrangian strain in the radial direction ranging from at least 0.01 to at most 50 MPa; preferably ranging from 0.05 to 10.0 MPa; more preferably ranging from 0.1 to 1.0 MPa; most preferably ranging from 0.6 to 0.9 MPa. In some embodiments the prosthesis is characterized in that the outer conduit has a Lagrangian strain in the circumferential direction ranging from at least 0.01 to at most 50 MPa; preferably ranging from 0.02 to 10.0 MPa; more preferably ranging from 0.04 to 1.0 MPa; most preferably ranging from 0.05 to 0.3 MPa. In some embodiments the prosthesis is characterized in that the outer conduit has a Lagrangian strain in the radial direction ranging from at least 0.01 to at most 50 MPa; preferably ranging from 0.05 to 10.0 MPa; more preferably ranging from 0.1 to 1.0 MPa; most preferably of about 0.4 MPa. In some embodiments the prosthesis is characterized in that the T-shaped conduit has a Lagrangian strain in the circumferential direction ranging from at least 0.01 to at most 50 MPa; preferably ranging from 0.02 to 10.0 MPa; more preferably ranging from 0.04 to 1.0 MPa; most preferably ranging from 0.05 to 0.3 MPa. In some embodiments the prosthesis is characterized in that the T-shaped conduit has a Lagrangian strain in the radial direction ranging from at least 0.01 to at most 50 MPa; preferably ranging from 0.05 to 10.0 MPa; more preferably ranging from 0.1 to 1.0 MPa; most preferably of about 0.7 MPa.

In some embodiments the prosthesis is characterized in that the inner conduit has a swelling ratio ranging from at least 0 to at most 99 %. In some embodiments the prosthesis is characterized in that the outer conduit has a swelling ratio ranging from at least 10 to at most 99 %; preferably ranging from 20 to 98%; more preferably ranging from 50 to 97 %; most preferably ranging from 60 to 97%. In some embodiments the prosthesis is characterized in that the T-shaped conduit has a swelling ratio ranging from at least 10 to at most 99 %; preferably ranging from 20 to 98%; more preferably ranging from 50 to 97 %; most preferably ranging from 60 to 97%.

In some embodiments the inner conduit comprises a fibrous network comprising microfibers and/or nanofibers, wherein the diameter of the fibers ranging from at least 0.01 to at most 5.0 µm; preferably ranging between 0.05 and 3.5 µm; more preferably ranging between 0.1 and 2.0 µm; most preferably ranging between 0.5 and 1.5 µm. In some embodiments the outer conduit comprises a fibrous network comprising microfibers and/or nanofibers, wherein the diameter of the fibers ranging from at least 0.01 to at most 5.0 µm; preferably ranging between 0.05 and 3.5 µm; more preferably ranging between 0.1 and 2.0 µm; most preferably ranging between 0.5 and 1.5 µm. In some embodiments the T-shaped conduit comprises a fibrous network comprising microfibers and/or nanofibers, wherein the diameter of the fibers ranging from at least 0.01 to at most 5.0 µm; preferably ranging between 0.05 and 3.5 µm; more preferably ranging between 0.1 and 2.0 µm; most preferably ranging between 0.5 and 1.5 µm.

In some embodiments the internal diameter of the inner conduit ranges between at least 1 and at most 40 mm; preferably between 5 and at most 35 mm; more preferably between 10 and at most 25 mm; most preferably is about 18 mm. In some embodiments the internal diameter of the outer conduit ranges between at least 1 and at most 40 mm; preferably between 5 and at most 35 mm; more preferably between 10 and at most 25 mm; most preferably is about 18 mm. In some embodiments the internal diameter of the T-shaped conduit ranges between at least 1 and at most 40 mm; preferably between 5 and at most 35 mm; more preferably between 10 and at most 25 mm; most preferably is about 18 mm. In some embodiments the difference between the internal diameter of the inner and outer conduits is at most 5 mm; preferably at most 1 mm; more preferably at most 0.1; most preferably less than 0.1 so that the diameter of the inner and outer diameter is essentially the same. In preferred embodiments the outer conduit and the T-shaped conduit have essentially the same internal diameter.

In some embodiments the length of the inner conduits ranges between at least 5 and at most 40 mm; preferably between 8 and at most 35 mm; preferably between 12 and at most 30 mm; most preferably between 15 and at most 25 mm. In some embodiments the length of the outer conduits ranges between at least 20 and at most 125 mm; preferably between 30 and at most 100 mm; preferably between 40 and at most 85 mm; most preferably between 50 and at most 75 mm. In preferred embodiments the length of the inner is shorter than the length of the outer conduit. In preferred embodiments the difference between the length of the inner conduit and the length of the outer conduit is at least 25 mm and at most 55 mm; preferably between 35 to 45 mm; most preferably around 40 mm.

The circumferential diameter refers to a circular diameter wherein the protrusions will fit. In some embodiments the external circumferential diameter of the protrusions ranges between at least 5 and at most 40 mm; preferably between 8 and at most 35 mm; preferably between 12 and at most 30 mm; most preferably between 25 and at most 35 mm. In some embodiments the circumferential commissure is coplanar with the most proximal end of the protrusions.

In some embodiments the inner conduit has a wall thickness ranging between at least 0.01 and at most 1.00 mm; preferably between 0.03 and at most 0.50 mm; more preferably between 0.05 and at most 0.3 mm; most preferably is about 0.1 mm or about 0.2 mm. In some embodiments the outer conduit has a wall thickness ranging between at least 0.01 and at most 2.00 mm; preferably between 0.05 and at most 1.50 mm; more preferably between 0.3 and at most 1.0 mm or at most 1.2 mm. In certain embodiments, the thickness of the outer conduit is 0.8 mm above the sinuses (i.e. towards the distal end) and 1.1 mm below the sinuses (i.e. towards the proximal end) to minimize the flexure of the outer conduit due to the movement of the inner conduit. In some embodiments the T-shaped conduit has a wall thickness ranging between at least 0.01 and at most 2.00 mm; preferably between 0.05 and at most 1.50 mm; more preferably between 0.3 and at most 1.0 mm; most preferably is about 0.3 mm. In preferred embodiments the outer conduit and the T-shaped conduit have essentially the wall thickness. In some embodiments the inner conduit has at least one region having a different wall thickness at the distal end compared to the wall thickness at the proximal end. In preferred embodiments the inner conduit has at least one region having a higher wall thickness at the distal end compared to the wall thickness at the proximal end; preferably the wall thickness is at least 0.01 mm thicker; more preferably the wall thickness is about 0.03 mm thicker; most preferably the wall thickness is about 0.05 mm thicker. This wall thickness difference corresponds to a percent difference of preferably at least 10%; more preferably about 25%; most preferably about 50%.A different wall thickness may prove beneficial for better valve functionality, decreasing the chance of fluid leakage towards the distal end. Additionally, a different wall thickness may allow an easier disposition of the inner conduit within the outer conduit. In some embodiments the inner conduit has at least one region having a higher wall thickness at the proximal end compared to the wall thickness at the distal end.

In some embodiments the inner conduit displays anisotropic fiber orientation, wherein the anisotropic ratio is at least 2:1, preferably at least 5:1; more preferably at least 10:1; most preferably about 20:1.In some embodiments the outer conduit displays anisotropic fiber orientation, wherein the anisotropic ratio is at least 2:1, preferably at least 5:1; most preferably about 10:1. In some embodiments the T-shaped conduit displays anisotropic fiber orientation, wherein the anisotropic ratio is at least 2:1, preferably at least 5:1; most preferably about 10:1. Anisotropy effects on tensile properties of fibers may cause variation in tensile toughness depending on fiber orientation and strain rate, wherein a higher rate of anisotropy may increase the tensile strength and elastic modulus of an object produced using said fibers.

In some embodiments the inner conduit has a porosity ranging from at least 20 to at most 99 %; preferably ranging from 40 to 96%; more preferably ranging from 50 to 93 %; most preferably ranging from 60 to 90%. In some embodiments the outer conduit has a porosity ranging from at least 20 to at most 99 %; preferably ranging from 40 to 96%; more preferably ranging from 50 to 93 %; most preferably ranging from 60 to 90%. In some embodiments the T-shaped conduit has a porosity ranging from at least 20 to at most 99 %; preferably ranging from 40 to 96%; more preferably ranging from 50 to 93 %; most preferably ranging from 60 to 90%. In preferred embodiments the outer conduit and the T-shaped conduit have essentially the same porosity.

In some embodiments the inner conduit has a pore size ranging between at least 0.05 and at most 15.0 µm; preferably between 0.1 and at most 10.0 µm; preferably between 0.2 and at most 8.0 µm; most preferably between 0.3 and at most 5.0 µm. In some embodiments the outer conduit has a pore size ranging between at least 1.0 and at most 90.0 µm; preferably between 10.0 and at most 75.0 µm; preferably between 15.0 and at most 50.0 µm; most preferably between 20.0 and at most 40.0 µm. In some embodiments the T-shaped conduit has a pore size ranging between at least 1.0 and at most 90.0 µm; preferably between 10.0 and at most 75.0 µm; preferably between 15.0 and at most 50.0 µm; most preferably between 20.0 and at most 40.0 µm. In preferred embodiments the outer conduit and the T-shaped conduit have essentially the same pore size.

In some embodiments the inner conduit has a permeability ranging between at least 0 and at most 20 mL cm⁻² min⁻¹. In some embodiments the outer conduit has a permeability ranging between at least 0.1 and at most 20 mL cm⁻² min⁻¹; preferably between 1 and at most 10.0 mL cm⁻² min⁻¹; more preferably between 2.5 and at most 7.5 mL cm⁻² min⁻¹; most preferably is about 5 mL cm⁻² min⁻¹. In some embodiments the T-shaped conduit has a permeability ranging between at least 0.1 and at most 20 mL cm⁻² min⁻¹; preferably between 1 and at most 10.0 mL cm⁻² min⁻¹; more preferably between 2.5 and at most 7.5 mL cm⁻² min⁻¹; most preferably is about 5 mL cm⁻² min⁻¹. In preferred embodiments the outer conduit and the T-shaped conduit have essentially the same permeability.

In a second aspect of the invention, the invention comprises a mandrel for electrospinning a vascular valved prosthesis as described herein according to the present invention.

The mandrel (600) may comprise multiple components, wherein at least a part of the mandrel (600) is configured to collapse with relation to the electrospun vascular valved prosthesis (100). The terms "collapse", "collapsible" or "collapsing" as used herein refer to the technical feature of the mandrel wherein at least a part of the mandrel may be folded or broken down inward upon removal of at least one means of fixation. The mandrel according to the invention in essence is composed of or at least comprises a central core, which typically is cylindrically shaped, and several (i.e. at least two) shell pieces which together may form a cylindrical shaft or tube which surrounds at least part of the mandrel core.

Also envisaged is the use of two half mandrel core pieces (i.e. a split mandrel core), to which the left and right ends of shell pieces may be respectively fixed. In such embodiment, the mandrel core does not extend through the entire interior of the shell pieces, but rather serves to affix the left and right ends of the shell pieces. In these embodiments, the mandrel core needs not necessarily be smaller in diameter than the shell pieces (when assembled), but may merely serve as a means to attach the shell pieces. In any case, detaching the shell pieces from the mandrel core allows the shell pieced to collapse.

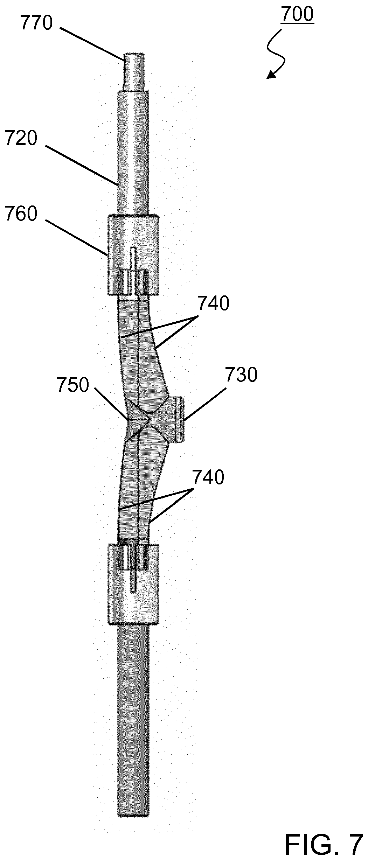

The shell pieces may be affixed to the core with fixation means (one or more fixation means may secure each shell piece onto the mandrel core). At least due to the thickness of the shell pieces, the composed mandrel has a certain diameter, which is larger than the diameter of the mandrel core. A prosthesis may be electrospun onto and surrounding the shell pieces. The mandrel is configured such that the fixation means which secure the shell pieces to the mandrel core may be loosened or removed, such that the shell pieces become detached from the mandrel core. To remove the mandrel from the electrospun prosthetic, the mandrel core is mechanically removed from the shell pieces. This allows to remove the mandrel core for instance by sliding out of the surrounding shell pieces, which during sliding may remain in place and hence do not exert any friction onto a prosthesis which has been formed around the shell. Once the core is removed, the remaining support for the shell pieces is lost, such that they may collapse inwardly. Then, by their design, the shell pieces collapse and can be removed from the electrospun part without friction with the prosthetic. The shell pieces and protrusions are fixed to the mandrel core by connectors that are slid or placed into a groove. The groove is configured to allow removal of the mandrel core after electrospinning of the prosthetic. When the mandrel core is removed, the shell pieces and the connectors collapsed according to their design. By collapsing, the shell pieces do not form or at least do not form a firm contact with the surrounding electrospun prosthesis, such that they can easily be removed from the prosthesis without any friction or causing friction-based damage. The assembled mandrel may typically have a generally cylindrical appearance (i.e., the assembled shell pieces), or be composed of subsections having a generally cylindrical appearance (e.g., a mandrel for creating bifurcated prostheses). It is to be understood however, that "cylindrical" encompasses geometries which are not mathematically exact cylinders, but roughly correspond to a cylinder (see e.g., figure 7).

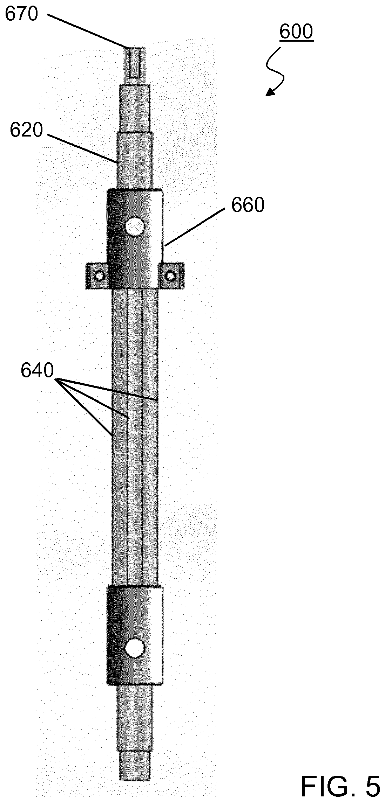

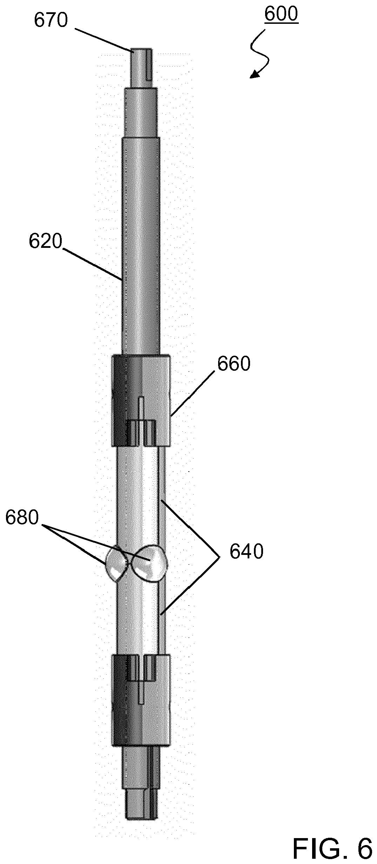

Exemplary mandrels may be found in figures 5, 6 and 7 which show a mandrel (600, 700) for electrospinning an inner conduit, an outer conduit and a T-shaped conduit, respectively. Specifically, the mandrel (600) used for electrospinning the inner conduit, cfr. figure 5, comprises one mandrel core (620) encapsulated by at least three, preferably four, radially inward collapsible shell pieces (640) which are attached using two fixation means (660) of which the top one further comprises a electrospinning set-up connector means (670) to connect the mandrel to an electrospinning setup. The mandrel (600) used for electrospinning the outer conduit, cfr. figure 6, comprises one mandrel core (620) encapsulated by at least two radially, preferably four, inward collapsible shell pieces (640) which further comprise at least two protrusions (650) and are attached using two fixation means (660). The mandrel (700) used for electrospinning the T-shaped conduit, cfr. figure 7, comprises a mandrel core arranged to resemble a bifurcation (750) and is encapsulated by at least four radially inward collapsible shell pieces (740) that are affixed using two fixation means (760); additionally, opposite the bifurcation (750) a mandrel trunk (730) is foreseen that may serve as a future attachment point with an electrospun outer conduit.

Hereto, according to the invention the mandrel for electrospinning comprises a cylindrical mandrel core, one or more fixation means, and two or more shell pieces; wherein said fixation means are configured for attaching said or more shell pieces on said mandrel core such as to form a cylindrical mandrel shell circumferentially encapsulating said mandrel core; and said mandrel core and/or said fixation means are configured for being removable by sliding out of or over said mandrel, preferably upon loosening of the fixation means; and said two or more shell pieces are configured for collapsing radially inward upon mandrel core and/or fixation means removal from said mandrel.

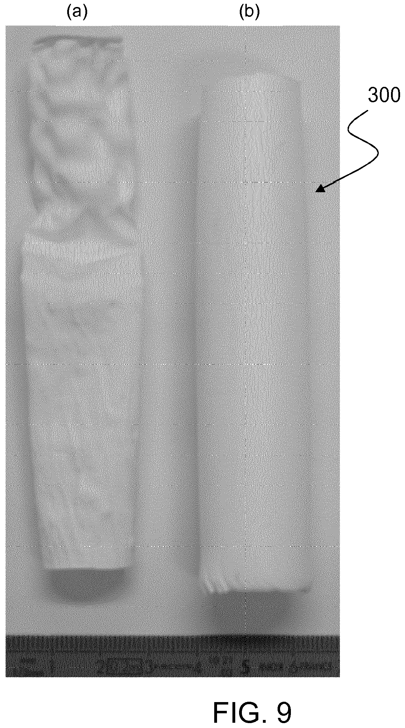

One major advantage of having a mandrel encapsulated by loose shell pieces attached using fixation means is that the shell pieces may be detached with the prosthesis surrounding the mandrel. The detachment of shell pieces makes the detachment of the prosthesis easier, reliable, and faster leading with increased production times. By having a mandrel encapsulated by collapsing shell pieces attached using a fixation means, the shell pieces may collapse inward within the prosthesis surrounding the mandrel. This in turn prevents at least partially or avoids completely structural damage to the prosthesis that may occur during detachment from the mandrel by excessive mechanical force, strain from expansion, and/or human error. Since these potential deformations upon detachment may degrade the structural and material integrity of the prosthesis, the reliability and reproducibility of a prosthesis manufactured using said mandrel with collapsible shell pieces may be greatly improved in comparison. Thus, a mandrel encapsulated by collapsing shell pieces may make detachment of the prosthesis even easier and more reliable, resulting in a prosthesis of improved quality. An example of a conduit electrospun using a mandrel with collapsible pieces may be found in figure 9 (b); this is in sharp contrast with a conduit electrospun using a non-collapsible mandrel according to a prior art method found in figure 9 (a) which did not allow easy removal of the prosthesis, resulting in a forced removal causing the prosthesis to fumble up and form folds along its surface.

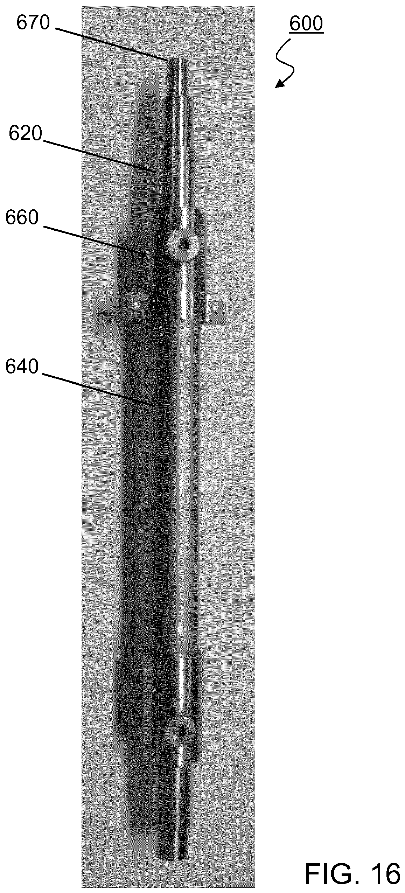

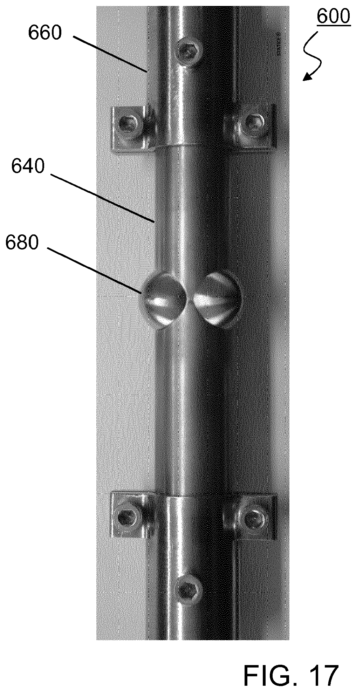

To better clarify this technical feature, figures 16 and 17 depict a mandrel (600) with collapsible shell pieces (640) and protrusions (650). As can be inferred, the mandrel core (620) is encapsulated by at least two shell pieces (640) that are stacked against each other; said shell pieces (640) are affixed to the core (620) by two fixation means (660) that stabilize and lock the mandrel components together. Optionally, on figure 17 the three shell pieces (640) further comprise three (650) protrusions attached to each shell pieces (640) using a protrusion connector (685). After electrospinning, the fixation (660) may be loosened or removed so that the mandrel core (620) can slide out from within the shell pieces (640); as a consequence, the remaining mandrel components, i.e., shell pieces (640) and optional protrusions (650), will collapse inward allowing the electrospun prosthesis (100) to be removed without causing damage. An example of a conduit electrospun using the above described mandrel may be found in figure 9 (b); this in sharp contrast with a conduit electrospun using a non-collapsible mandrel according to a prior art method found in figure 9 (a) which did not allow easy removal of the prosthesis, resulting in a forced removal causing the prosthesis to fumble up and form folds along its surface.

In some embodiments the shell pieces are fixed mechanically by connectors; preferably the connectors provide no friction on the mandrel core when fixed or removed. In some embodiments the shell pieces can be interchanged with shell pieces of different dimensions, i.e., size and width. In some embodiments the shell pieces can be interchanged with shell pieces of greater dimensions. In some embodiments the shell pieces can be interchanged with shell pieces of smaller dimensions. In some embodiments, one or more of the shell pieces may comprise shell piece sections. Such may be advantageous for instance if protrusions on the mandrel are to be provided, as depicted in figure 6, or for a bifurcated mandrel, as depicted in figure 7.

An advantage of having a mandrel encapsulated by multiple, interchangeable shell pieces, instead of a singular one-piece mandrel, is the level of customization towards the desired prosthesis dimensions and components. By interchanging shell pieces with wider or narrower variants, the diameter of the prosthesis can be easily adjusted, without the need to produce a completely new mandrel. This translates into increased reliability, customization, and ease of production.

In some embodiments the mandrel comprises one or more radially extending protrusions, wherein said protrusions are configured for collapsing radially inward upon mandrel core and/or fixation means removal from said mandrel.

In some embodiments the mandrel comprises an electrospinning set-up connector means said connector means is configured for attachment to an electrospinning system by a person skilled in the art. In alternative embodiments, the electrospinning set-up connector means may be detached from the mandrel core, thereby obtaining a higher degree of compatibility with electrospinning systems. Should an electrospinning system be modified for different means of rotation, or attachment, the fixation means comprising the electrospinning set-up connector means may be replaced without having to replace the mandrel. This in turn makes the mandrel easier to use and more compatible with advances in the art of electrospinning.

In some embodiments the mandrel is for electrospinning the inner conduit. In some embodiments the mandrel is for electrospinning the outer conduit. In some embodiments the bifurcated mandrel is for electrospinning the T-shaped conduit. In some embodiments the mandrel is for electrospinning the outer conduit comprising protrusions. In some embodiments the mandrel is for electrospinning a prosthesis. In some embodiments the mandrel is for electrospinning a vascular prosthesis. In some embodiments the mandrel is for electrospinning a vascular valved prosthesis. In some embodiments the mandrel is for electrospinning a biocompatible vascular valved prosthesis. In preferred embodiments the mandrel is for electrospinning a vascular valved prosthesis suitable for pulmonary valve replacement or aortic valve replacement. In preferred embodiments the mandrel is for electrospinning a biodegradable vascular valved prosthesis.

A mandrel with collapsible protrusions may have advantages similar to the above described advantages of a mandrel with collapsible shell pieces. Mainly, any structural damage to the prosthesis during detachment from the mandrel may be avoided at least partially to completely. Since these potential deformations upon detachment may degrade the structural and material integrity of the prosthesis, the reliability and reproducibility of prosthesis manufactured using said mandrel with collapsible shell pieces may be greatly improved in comparison. Thus, a mandrel encapsulated by collapsing shell pieces and collapsing protrusion may make detachment of the prosthesis even easier and more reliable, resulting in a prosthesis with protrusion of improved quality. An exemplary mandrel assembled according to this embodiment is shown in figure 17. Protrusions may be mounted on the mandrel core through connectors. The protrusions and/or connectors may for instance be inserted (e.g. slid) into the mandrel core through longitudinal slits or slots; whereas the shell pieces may be subsequently mounted and connected to the mandrel core and/or protrusions via fixation means (e.g. screws or connector pins). In case of such protrusions, advantageously each shell piece may be composed of a "left" and "right" shell piece section. The skilled person will understand that alternative arrangements may also be employed. For instance, the protrusions may be connected to the shell pieces prior to mounting on the mandrel core, as long as ultimately, removal or loosening of the fixation means allows the mandrel core and/or fixation means to be removed, after which the shell pieces collapse and can be removed from the prosthesis.

In some embodiments the diameter of the mandrel shell is at least 5% larger than the diameter of said mandrel core. In preferred embodiments the diameter of the mandrel shell is at least 10% larger than the diameter of said mandrel core. In some embodiments the bifurcated mandrel comprises at least one bifurcation. In preferred embodiments the bifurcated mandrel is T-shaped. In some embodiments the bifurcated mandrel comprises a mandrel trunk which is affixed through a sliding motion around or into said mandrel core such as to obtain a bifurcation, preferably a T-shape. The advantage of having an affixed mandrel trunk is that the mandrel can be easily customized for electrospinning a tubular prosthesis or a prosthesis comprising a bifurcation. This gives the mandrel a higher level of compatibility, and increases the ease of use of said mandrel. Preferably, the (central) axis of the mandrel trunk is orthogonal or substantially orthogonal to the (central) axis of the mandrel core. The mandrel trunk (or mandrel trunk core) may have an opening through which the mandrel core may be inserted, such as to create a bifurcation. As with the embodiments of the mandrel discussed herein elsewhere, a bifurcated mandrel comprises shell pieces encapsulating the mandrel (trunk) core, and which shell pieces can be affixed to the mandrel core as described herein elsewhere. According to certain embodiments, the mandrel trunk is also composed of a mandrel trunk core, encapsulated in mandrel trunk shell pieces, which may be affixed as described herein elsewhere.

It is preferred that the mandrel be at least partially manufactured from or comprise electroconductive material, such as for instance a metal or metal alloy. Examples of preferential metallic materials include aluminum or stainless steel (cfr. AISI 303, AISI 316L). It is to be understood that the mandrel should at least be suitable for electrospinning, such that mandrels comprised of at least partially comprising materials not suitable for electrospinning are not envisaged according to the present invention. In certain embodiments, at least the shell pieces comprise or are composed of electro conductive material. In a further aspect, the invention relates to a kit of parts comprising the individual components constituting the mandrel according to the invention as defined herein. Hereto, such kit may comprise a mandrel core, two or more shell pieces, and one or more fixation means, whereby each of the components are configured for assembly of the mandrel according to the invention as defined herein.

In a third aspect of the invention, the invention comprises the use of the mandrel for electrospinning, such as electrospinning a (biodegradable) prosthesis, such as a vascular valved prosthesis according to certain embodiments of the present invention.

In some embodiments the use of the mandrel may be purposed for electrospinning a prosthesis. In some embodiments the use of the mandrel may be purposed for electrospinning a vascular prosthesis. In some embodiments the use of the mandrel may be purposed for electrospinning a (biocompatible) vascular valved prosthesis.

In some embodiments the use of the mandrel may be purposed for electrospinning a biocompatible prosthesis. In some embodiments the use of the mandrel may be purposed for electrospinning a biocompatible vascular prosthesis. In preferred embodiments the use of the mandrel may be purposed for electrospinning a biocompatible vascular valved prosthesis.

In some embodiments the use of the mandrel may be purposed for electrospinning a biodegradable prosthesis. In some embodiments the use of the mandrel may be purposed for electrospinning a biodegradable vascular prosthesis. In preferred embodiments the use of the mandrel may be purposed for electrospinning a biodegradable vascular valved prosthesis.

In some embodiments the use of the mandrel may be purposed for electrospinning the prosthesis, according to an embodiment of the present invention.

In a fourth aspect of the invention, the invention comprises the method for manufacturing a vascular valved prosthesis according to an embodiment of the present invention.

In some embodiments the method for manufacturing a vascular prosthesis comprises the step of electrospinning a vascular valved prosthesis, preferably a prosthesis according to the invention as described herein.

In some embodiments the method for manufacturing a vascular prosthesis comprises the step of electrospinning a prosthesis. In some embodiments the method for manufacturing a vascular prosthesis comprises the step of electrospinning a vascular prosthesis. In preferred embodiments the method for manufacturing a vascular prosthesis comprises the step of electrospinning a vascular valved prosthesis.

In some embodiments the method for manufacturing a biocompatible vascular prosthesis comprises the step of electrospinning a biocompatible prosthesis. In some embodiments the method for manufacturing a biocompatible vascular prosthesis comprises the step of electrospinning a biocompatible vascular prosthesis. In preferred embodiments the method for manufacturing a biocompatible vascular prosthesis comprises the step of electrospinning a biocompatible vascular valved prosthesis.

In some embodiments the method for manufacturing a biodegradable vascular prosthesis comprises the step of electrospinning a biodegradable prosthesis. In some embodiments the method for manufacturing a biodegradable vascular prosthesis comprises the step of electrospinning a biodegradable vascular prosthesis. In preferred embodiments the method for manufacturing a biodegradable vascular prosthesis comprises the step of electrospinning a biodegradable vascular valved prosthesis.

In preferred embodiments the method for manufacturing a vascular valved prosthesis comprises the step of electrospinning a vascular valved prosthesis suitable for pulmonary valve replacement or aortic valve replacement. In preferred embodiments the method for manufacturing a biocompatible vascular valved prosthesis comprises the step of electrospinning a biocompatible vascular valved prosthesis suitable for pulmonary valve replacement or aortic valve replacement. In preferred embodiments the method for manufacturing a biodegradable vascular valved prosthesis comprises the step of electrospinning a biodegradable vascular valved prosthesis suitable for pulmonary valve replacement or aortic valve replacement.

In some embodiments the method for manufacturing a vascular valved prosthesis, preferably wherein said vascular valved prosthesis is suitable for pulmonary valve replacement or aortic valve replacement, comprises the steps of (1) electrospinning an inner conduit having a distal end and a proximal end; (2) electrospinning an outer conduit having a distal end and a proximal end, and optionally comprising three coplanar radially equidistally spaced outward protrusions; (3) attaching the proximal end of said inner conduit circumferentially along the inside of the outer conduit towards the proximal end of the outer conduit to form a circumferential commissure; (4) attaching the distal end of said inner conduit at two or more, preferably three equidistally spaced discrete, optionally longitudinal, positions to the inside of the outer conduit, optionally along longitudinal lines separating the protrusions; (5) electrospinning a T-shaped conduit having a trunk and lateral arms extending therefrom; (6) attaching the distal end of said outer conduit to the trunk of said T-shaped conduit; (7) wherein said inner conduit is attached to the outer conduit such as to function as a valve allowing unidirectional flow of a fluid through said outer conduit from the outer conduit's proximal to distal end.

It is to be understood that the order of the steps in the method above may be changed, e.g., the T-shaped conduit may be attached to the outer conduit prior to attaching the inner conduit to the outer conduit. In certain embodiments, steps (2) and (5) of the method may be combined, such as to create in one step a T-shaped outer conduit. In this way there is no need for separately attaching the bifurcation to the outer conduit.

The method for manufacturing a biocompatible vascular valved prosthesis requires steps (1), (2) and (5) to use a biocompatible material, preferably from the list provided herein. Optionally, this method may introduce an additional step (8) to improve the biocompatibility, such as providing a biocompatible coating or functionalization with biologically active compounds.

The method for manufacturing a biodegradable vascular valved prosthesis requires steps (1), (2) and (5) to use a biocompatible material, preferably from the list provided herein. Optionally, this method may introduce an additional step (8) to improve the biodegradability, such as providing a coating to promote an improved level of exogenous cell seeding and tissue regeneration.

In a further aspect, the invention relates to a prosthesis as described herein, obtained or obtainable with the mandrel and/or the methods according to the invention as described herein.

### Examples

### Example 1: Properties and dimensions of an electrospun vascular valved prosthesis.

A vascular valved prosthesis as described in the present invention may be manufactured with different parameters and may thus be obtained in different dimensions. In an example, a T-shaped vascular valved prosthesis with a diameter of about 18 mm comprises an inner, outer and T-shaped conduit affixed together through an attachment. To better illustrate the example, a reference is made to figures 11 and 12, which demonstrate a tubular vascular valved prosthesis from several perspectives; and figures 13, 14 and 15, which similarly demonstrate a T-shaped vascular valved prosthesis.

The inner conduit of the T-shaped vascular valved prosthesis with a diameter of about 18 mm displays the following properties:
- The internal diameter of the inner conduit is 18 mm.
- The length of the inner conduit is 20 mm.
- The thickness of the inner conduit is between 0.05 and 0.3 mm, preferably around 0.1 mm.
- The linear elastic regimen between 40% to 60%.
- The Young modulus in the circumferential direction is between 5 to 100 Mpa.
- The Young modulus in the radial direction is between 0.5 to 15 Mpa.
- The Lagrangian strain in the circumferential direction is between 0.1 to 0.4 Mpa.
- The Lagrangian strain in the radial direction is between 0.6 to 0.9 Mpa.
- The size of the pores ranges between 0.3 to 5 µm.
- The porosity is at least 60% to at most 90%.
- The anisotropy ratio is about 20:1.

The outer conduit with protrusions of the T-shaped vascular valved prosthesis with a diameter of about 18 mm displays the following properties:
- The internal diameter of the outer conduit is 18 mm.
- The length of the outer conduit is 63 mm.
- The thickness of the outer conduit is between 0.3 and 1 mm, preferably around 0.6 mm.
- The circumferential diameter of the outer conduit with protrusions is 28 mm.
- The Young modulus for both circumferential and radial direction is between 0.01 to 0.5 MPa.
- The Lagrangian strain in the circumferential direction is between 0.05 to 0.3 Mpa.
- The Lagrangian strain in the radial direction is about 0.4 Mpa.
- The swelling ratio is between 60 and 97%.
- The size of the pores ranges between 20 to 40 µm.
- The porosity is at least 60% to at most 90%.
- The permeability is about 5 mL cm⁻² min⁻¹.
- The anisotropy ratio is about 10:1.

The T-shaped conduit of the T-shaped vascular valved prosthesis with a diameter of about 18 mm displays the following properties:
- The internal diameter of the proximal end of the T-shaped conduit is 18 mm.
- The internal diameter of the distal end of the T-shaped conduit is 12 mm.
- The vertical length of the T-shaped conduit is 23 mm.
- The horizontal length of the T-shaped conduit is 96 mm.
- The thickness of the T-shaped conduit is between 0.3 and 1 mm, preferably around 0.6 mm.
- The Young modulus for both circumferential and radial direction is 0.01 to 0.5 MPa.
- The Lagrangian strain in the circumferential direction is between 0.05 to 0.3 Mpa.
- The Lagrangian strain in the radial direction is about 0.7 Mpa.
- The swelling ratio is between 60 and 97%.
- The size of the pores ranges between 20 to 40 µm.
- The porosity is at least 60% to at most 90%.
- The permeability is about 5 mL cm⁻² min⁻¹.
- The anisotropy ratio is about 10:1.

The outer and T-shaped conduits internal diameter and thickness should be essentially the same. The conduits of the prosthesis are made from an electrospun fibrous network which comprises nanofibers and/or micro fibers with a fiber diameter ranging from 0.5 to 1.5 µm; preferably the diameter of the fibers is about 1 µm. By changing the orientation of the fibers the porosity of the inner and outer conduit can be controlled. This way, the inner conduit may be processed by multilayer electrospinning, wherein 2 to 3 distinct layers are deposited, each layer ranging between 20 to 50 µm.

As a result of this variation in material ratio and processing parameters, the conduits differ in their material properties and biodegradability rate. Concretely, the inner conduit supports the vascular structure with a persistence of mechanical properties for 24 months; while the outer and T-shaped conduits support the vascular structure with a persistence of mechanical properties for 6 to 12 months.

The figures further demonstrate how the inner conduit and the outer conduit may be affixed; thus forming a tubular, vascular valved prosthesis. Specifically, figure 11 shows the attachments from the outside in a frontal view; while figure 12 provides a top view to observe the attachments from the inside. Said attachment may be performed using a variety of methods, such as sutures, staples, glue, welds (laser, vibration, ultrasonic, induction, high frequency) or a combination thereof; however, for this example sutures were used. Next, the combined inner and outer conduit may be affixed in a similar manner to the T-shaped conduit, as is demonstrated in figure 13 from the front, in figure 14 from the side and in figure 15 from the bottom; thus forming a T-shaped, vascular valved prosthesis.

Example 2. Properties and dimensions of a mandrel for electrospinning a vascular prosthesis.

Figures 6 and 17 demonstrate a mandrel (600) for electrospinning an outer conduit comprising the following dimensions:
- The cylindrical mandrel core (620) has a diameter of 14 mm.
- The cylindrical mandrel core (620) encapsulated by shell pieces has a diameter of 18 mm.
- The upper and lower fixation means (660), which are affixed to the cylindrical mandrel core (620) encapsulated by shell pieces (640), each have a diameter of 22 mm.
- The upper and lower fixation means (660) each have a length of 40 mm.
- The shell pieces (640) each have a length of 150 mm.
- The operable electrospinning distance on the shell pieces situated between the upper and lower fixation means (660) is 120 mm.
- The cylindrical mandrel core has a length of 295.5 mm.
- The upper and lower fixation means (660) have a length of 40 mm.
- The upper and lower fixation means (660) have a length of 40 mm.

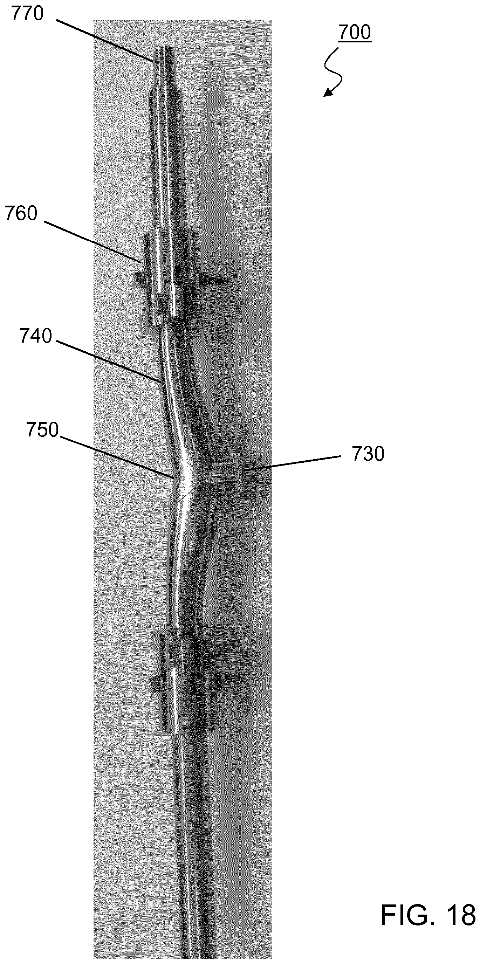

These dimensions are suitable for mandrels intended for electrospinning the inner conduit (cfr. figures 5 and 16) or T-shaped conduit (cfr. figures 7 and 18); yet, some parameters such as the length of the shell pieces and the fixation means may vary. Using the dimensions as described above, the mandrel is suitable for electrospinning the outer conduit with a diameter of 18 mm as described in example 1.

For reference, figure 9(b) shows a conduit which was electrospun with the mandrel of this example, compared to a conduit electrospun with a prior art mandrel, cfr. 9(a), i.e. a monolithic mandrel, not comprising collapsible shell pieces. It is clear that the mandrel according to the invention produces better quality prosthesis.

### Example 3. Method for the manufactory of a vascular valved prosthesis comprising a T-shaped bifurcation using a mandrel for electrospinning.

As an example of a method for manufactory of a T-shaped vascular valved prosthesis (100) using a mandrel for electrospinning we refer to figures 5-7 and 16-18. Respectively, figures 5 and 16 demonstrate a schematic and an assembly of mandrel (600) for electrospinning the inner conduit (200); figures 6 and 17 similarly demonstrate a mandrel (600) for electrospinning the outer conduit (300); and figures 7 and 18 similarly demonstrate a mandrel (700) for electrospinning the T-shaped conduit (400).

The vascular valved prosthesis comprising a T-shaped bifurcation is manufactured by comprising the following steps:
(1) Solubilizing a polymer in an appropriate solvent for electrospinning, preferably 1,1,1,3,3,3-Hexafluoro-2-propanol preferably with a weight/volume concentration between 5-15 % w/v.
(2) Electrospinning an inner conduit, an outer conduit and a T-shaped conduit, wherein (i) said inner conduit having a distal end and a proximal end is electrospun using the mandrel shown in figures 5 and 16; (ii) said outer conduit having a distal end and a proximal end is electrospun using the mandrel shown in figures 6 and 17; and (iii) said T-shaped conduit having a distal end and a proximal end is electrospun using the mandrel shown in figures 7 and 18; steps (i) to (iii) may be performed simultaneously or in series, wherein the order of said steps has no discernible impact on the method.
(3) Attaching the proximal end of said inner conduit circumferentially along the inside of the outer conduit towards the proximal end of the outer conduit to form a circumferential commissure; the inner conduit can be fixed to the outer conduit by suture, stapling, gluing, welding (laser, vibration, ultrasonic, induction, high frequency) or a combination of the processes described.
(4) Attaching the distal end of said inner conduit at three equidistally spaced discrete positions to the inside of the outer conduit along longitudinal lines separating the protrusions.
(5) Attaching the distal end of said outer conduit to the trunk of said T-shaped conduit, wherein said inner conduit is attached to the outer conduit such as to function as a valve allowing unidirectional flow of a fluid through said outer conduit from the outer conduit's proximal to distal end; the outer conduit element can be fixed to the T-shaped conduit element by suture, stapling, gluing, welding (laser, vibration, ultrasonic, induction, high frequency) or a combination of the processes described.

Optionally, based on the functionality of said vascular valved prosthesis comprising a T-shaped bifurcation the method of manufactory may further comprise the following steps:
(7) Functionalization of the device with biologically active compounds.
(8) Sterilization of the device.

The following electrospinning parameters were used to manufacture the inner conduit (200) using a mandrel (600) assembled for electrospinning an inner conduit:
- Flowrate: 10 mL/h.
- Electro-spinning distance: 120 mm.
- Voltage: + 12 kV.
- Mandrel velocity: 1000 rpm.
- Raster max speed: 30 mm/s.

The following electro-spinning parameters were used to manufacture the outer conduit using a mandrel (600) assembled for electrospinning an outer conduit:
- Flowrate: 10 mL/h.
- Electro-spinning distance: 120 mm.
- Voltage: + 12 kV.
- Mandrel velocity: 1000 rpm.
- Raster max speed: 30 mm/s.

The following electro-spinning parameters were used to manufacture the T-shaped conduit using a bifurcated mandrel (700) assembled for electrospinning a T-shaped conduit:
- Flowrate: 10 mL/h.
- Electro-spinning distance: 100-150 mm.
- Voltage: + 12 kV.
- Mandrel velocity: 1000 rpm.
- Raster max speed: 30 mm/s.

Figures 13 to 15 demonstrate a vascular valved prosthesis manufactured as described in this example from several views in perspective; the conduits were affixed using the methods described in example 1.

Figure 20 and 21 demonstrate the application of a reinforcement ring on prosthesis according to the invention. The Table below shows that the functionality of the valve over time is improved when a reinforcement is present.

Comparative analysis of the opening area of the valve with and without a ring after one hour of test

| **Valved tube** | **Effective opening area (%)** |
|---|---|
| without a reinforcement ring | 69 |
| with a bioresorbable reinforcement ring | 93 |

## Claims

1. A fully biodegradable vascular valved prosthesis, preferably allowing tissue regeneration and growth potential, comprising an electrospun inner conduit having a proximal end and a distal end and which is disposed within an electrospun outer conduit having a proximal end and a distal end, wherein the inner conduit is attached to the outer conduit such as to function as a valve allowing unidirectional flow of a fluid through said outer conduit from the outer conduit's proximal to distal end,
wherein the proximal end of said inner conduit is attached circumferentially along the inside of the outer conduit towards the proximal end of the outer conduit to form a circumferential commissure;
wherein the distal end of said inner conduit is attached to the inside of the outer conduit towards the distal end of said outer conduit to form focal commissures at one or more discrete positions, wherein the focal commissures may extend longitudinally from the distal end of the inner conduit towards the proximal end of the inner conduit.

2. The prosthesis according to claim 1, which is bifurcated, preferably T-shaped.

3. The prosthesis according to claim 1 or 2, wherein said outer conduit comprises at least one radially outward protrusion.

4. The prosthesis according to any of claims 1 to 3, wherein the outer conduit comprises a proximal circumferential reinforcement and/or wherein the inner conduit comprises a distal section having increased wall thickness compared to the proximal section.

5. The prosthesis according to any of claims 1 to 4 for pulmonary valve replacement or aortic valve replacement.

6. The prosthesis according to any of claims 3 to 5, wherein said outer conduit comprises three coplanar radially equidistally spaced outward protrusions, and wherein the distal end of said inner conduit is attached at three equidistally spaced discrete positions to the inside of the outer conduit along longitudinal lines separating the protrusions.

7. The prosthesis according to any of claims 2 to 6, wherein a T-shaped conduit is attached along the distal end of said outer conduit.

8. The prosthesis according to any of claims 1 to 7, wherein the inner conduit comprises one or more reinforcements, preferably at or near the commissures.

9. The prosthesis according to any of claims 1 to 8, wherein the outer conduit and if present the T-shaped conduit biodegrades faster than the inner conduit.
